(19) Europäisches Patentamt
European Patent Office
Office européen des brevets

(11) Veröffentlichungsnummer: **0 282 456**
**A2**

(12) **EUROPÄISCHE PATENTANMELDUNG**

(21) Anmeldenummer: **88810142.5**

(22) Anmeldetag: **07.03.88**

(51) Int. Cl.⁴: **C 07 D 493/22**
A 61 K 31/365, A 01 N 43/90,
A 23 K 1/17
//(C07D493/22,313:00,313:00,
311:00,307:00)

(30) Priorität: **13.03.87 CH 955/87**

(43) Veröffentlichungstag der Anmeldung:
**14.09.88 Patentblatt 88/37**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Maienfisch, Peter, Dr.**
**Traugott Meyer-Strasse 5**
**CH-4147 Aesch (CH)**

Die Bezeichnung der Erfindung wurde geändert (Richtlinien für die Prüfung im EPA, A-III, 7.3).

(54) **Milbemycin-Derivate zur Bekämpfung von Parasiten an Tieren.**

(57) Es werden neue 13-Vinyl-milbemycin-Derivate der Formel I

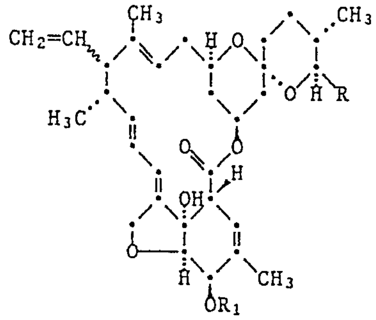

(I)

worin
R für Methyl, Ethyl, Isopropyl oder sek.-Butyl oder für die Gruppe $-\underset{CH_3}{C}=CH-A$ steht, wobei A Methyl, Ethyl oder Isopropyl bedeutet; und

$R_1$ Wasserstoff, eine Silyl- oder eine Acylgruppe darstellt; unter Einschluss ihrer Säureadditionssalze und Metallkomplexe sowie deren Herstellung und Verwendung gegen Parasiten an Nutztieren und gegen Schadinsekten beschrieben.

EP 0 282 456 A2

**Beschreibung**

Mittel zur Bekämpfung von Parasiten an Tieren

Die vorliegende Erfindung betrifft neue 13-Vinyl-Milbemycin-Derivate der nachstehenden Formel I, deren Herstellung, Mittel die mindestens eine dieser Substanzen als Wirkstoff enthalten sowie deren Verwendung zur Bekämpfung von Schädlingen wie Ekto- und Endoparasiten, insbesondere am Nutztier.

Bei den erfindungsgemässen Verbindungen handelt es sich um 13-Vinyl-Milbemycine der allgemeinen Formel I

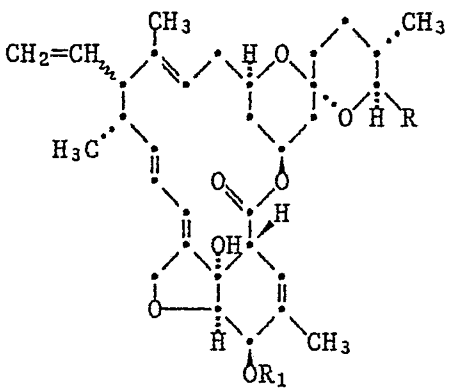

(I)

worin

R für Methyl, Ethyl, Isopropyl oder sek.-Butyl oder für die Gruppe 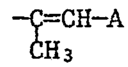 steht, wobei A Methyl, Ethyl oder Isopropyl bedeutet; und

$R_1$ Wasserstoff, eine Silyl- oder eine Acylgruppe darstellt; unter Einschluss ihrer Säureadditionssalze und Metallkomplexe.

Besonders bevorzugt sind die neuen Verbindungen der allgemeinen Formel I

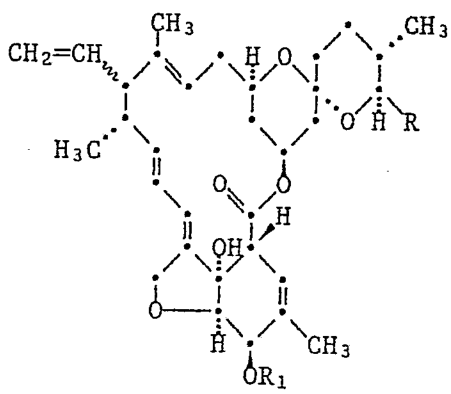

(I)

worin

R für Methyl, Ethyl, Isopropyl oder sek.-Butyl steht; und

$R_1$ Wasserstoff, eine Silyl- oder eine Acylgruppe darstellt; unter Einschluss ihrer Säureadditionssalze und Metallkomplexe.

Der Vinylsubstituent an C(13) kann dabei sowohl entweder in 13β-oder in 13α-Position stehen, oder es kann ein beliebiges Gemisch 13α/13β vorliegen.

Formel I repräsentiert somit Milbemycin-Abkömmlinge, die eine 13β-bzw. 13α-Vinylgruppe enthalten und in 5-Position acyliert oder silyliert sein können und in den Fällen in denen der Acylrest einen stickstoffhaltigen Heterocyclus trägt als Säureadditionssalz oder Metallkomplex vorliegen können.

Verbindungen der Formel I, worin $R_1$ Wasserstoff oder eine Acylgrupe bedeutet, sind bevorzugt. Silylgruppen als $R_1$ sind im allgemeinen als Schutzgruppen aufzufassen. Als geeignete Silylgruppen für $R_1$ kommt der Rest $-Si(R_2)(R_3)(R_4)$ in Frage, wobei $R_2$, $R_3$ und $R_4$ vorzugsweise unabhängig voneinader für $C_1$-$C_6$-Alkyl, Benzyl oder Phenyl stehen und beispielsweise eine der Gruppen Trimethylsilyl, Thexyldimethylsilyl (Thexyl = 1,1,2-Trimethylpropyl), Diphenyl-tert.-butylsilyl, bis(Isopropyl)methylsilyl oder Triphenylsilyl und

vorzugsweise tert.-Butyldimethylsilyl bilden.

Unter dem Begriff Acyl selbst oder als Bestandteil einer Acyloxygruppe sollen im Rahmen vorliegender Erfindung beispielsweise unsubstituiertes oder substituiertes Alkylcarbonyl (= Alkanoyl), Arylcarbonyl und Aralkylcarbonyl mit 1 bis 30 C-Atomen, vorzugsweise mit 1 bis 18 C-Atomen, insbesondere unsubstituiertes oder substituiertes Acetyl, Propionyl, Butyryl oder Benzoyl verstanden werden, die im Falle der Alkanoyle als Substituenten z.B. Halogen, Alkoxy, Haloalkoxy, Aryloxy, Hydroxy, Aryl, Aryloxy oder einen ungesättigten oder gesättigten, fünf- oder sechsgliedrigen, heterocyclischen Ring mit ein- bis drei Heteroatomen ausgewählt aus der Reihe Stickstoff, Sauerstoff und Schwefel enthalten können, wobei die als Aryl bezeichneten Teile ihrerseits durch Halogen, Cyano, Alkyl, Alkoxy, Haloalkyl, Haloalkoxy, Alkylthio und/oder Nitro substituiert sein können und z.B. für α-, β-Naphthyl oder vorzugsweise Phenyl stehen.

Aralkyl steht für einen über eine geradkettige oder verzweigte Alkylenbrücke verbundenen aromatischen Rest; der einfachste Vertreter ist die Benzylgruppe.

Unter dem Begriff Alkyl selbst oder als Bestandteil eines anderen Substituenten sind je nach Zahl der angegebenen Kohlenstoffatome beispielsweise folgende geradkettige Gruppen zu verstehen: Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl usw. sowie ihre verzweigten Isomeren, wie z.B. Isopropyl, Isobutyl, tert.-Butyl, Isopentyl usw.. Alkenyl steht selbst oder als Bestandteil eines Alkenyloxy z.B. für Propenyl-(1), Allyl, Butenyl-(1), Butenyl-(2), Butenyl-(3) usw. Alkinyl steht beispielsweise für Ethinyl, Propinyl-(1), Propargyl, Butinyl-(1), usw. Unter Halogen selbst oder als Vorsilbe Halo soll hier und im folgenden Fluor, Chlor, Brom oder Jod verstanden werden, vorzugsweise Chloro oder Brom. Beispiele für Cycloalkyl selbst oder als Bestandteil von Cycloalkoxy sind Cyclopropyl, Cyclobutyl, Cyclopentyl, Cyclohexyl, Cycloheptyl, Cyclooctyl etc.. Haloalkyl repräsentiert allein oder als Bestandteil von Haloalkoxy einen ein-bis perhalogenierten Alkylrest, wie z.B. $CH_2J$, $CH_2Br$, $CH_2Cl$, $CH_2F$, $CHCl_2$, $CHF_2$, $CCl_3$, $CBr_3$, $CF_3$, $C_2F_5$, $C_2Cl_5$, CFClBr, usw., wobei der Rest auch gleichzeitig durch unterschiedliche Halogenatome substituiert sein kann; bevorzugt ist jedoch der $CF_3$-Rest. Typische fünfgliedrige heterocyclische Ringe sind: Furan, Thiophen, Pyrrol, Isoxazol, Isothiazol, Furazan, Imidazol, 1,2,4-Triazol, 1,2,3-Triazol, Pyrazol, Pyrrolin, Oxazol, Thiazol, Thiadiazole, Pyrazolin, Thiazolin, Pyrazolidin, Pyrrolidin, Oxazolidin, Thiazolidin, Oxadiazol, Imidazolin, Imidazolidin, Pyrazolidin, Tetrahydrofuran; und typische sechsgliedgrige heterocyclische Ringe sind Pyridin, Pyridazin, Pyrimidin, Pyrazin, Thiazin, Thiadiazine, Pyrane, Piperidin, Piperazin, Morpholin, Perhydrothiazin, Dioxan sowie ihre teilhydrierten bzw. teilgesättigten Homologen, usw.. Oxo substituierte Heterocyclen sind hier und im folgenden insbesondere fünfgliedrige und sechsgliedrige Lactone und Lactame, wie z.B. Butyrolactone, Valerolactone, Butyrolactam, Valerolactam, aber auch bicyclische Systeme wie Camphan.

Beispiele für den heterocyclischen Substituenten sind Imidazol, Pyrazol, 1,2,3-Triazol, 1,2,4-Triazol, 1,3,4-Triazol oder Tetrazol sowie ein- bis zweifach durch $C_1$-$C_6$-Alkylreste substituierte Azole wie 2-Ethyl-4-methylimidazol, 2-Isopropylimidazol, Methylimidazol, 3,5-Dimethyltriazol, Ethyltriazol, 3,4-Diethylpyrazol u.a.

Nachfolgend werden einige typische Vertreter von $R_1$-Acylresten genannt, wobei diese Aufzählung keinen limitierenden Charakter besitzt: $COCH_3$, $COCH_2Cl$, $COCF_3$, $COCH_2Br$, $COCH_2F$, $COC_2H_5$, $COC_2Cl_5$, $COCH_2OCOCH_3$, $COCH_2OCOC_4H_9$(t), $COCH_2OCH_3$, $COCHFOCOCH_3$, $COCH(CH_3)OCOCH_3$, $COCH_2OCOC_2H_5$, $COCH_2OCOCH_2Cl$, $COCH_2OCOC_6H_4F$(3), $COCH_2OCOC_6H_4OCH_3$(3), $COCH_2OCH_2OCH_3$, $COCH_2OC_2H_5$, $COCH_2SCH_3$, $COCH_2OCOC_6H_4Cl$(3), $COCH_2$-(1H-1,2,4-triazol-1-yl), $COCH_2$-(2,3-dihydropyran-2-yl), $COCH_2$(1H-imidazol-1-yl) und $COCH_2OCO$-(pyrolidon-(2)-5-yl).

Salze, insbesondere solche mit guter physiologischer Verträglichkeit, werden aus Verbindungen der Formel I mit anorganischen oder organischen Säuren gebildet. Als Beispiele seien Chlorwasserstoffsäure, Bromwasserstoffsäure, Schwefelsäure, Salpetersäure, Phosphorsäure, Methansulfonsäure, p-Toluolsulfonsäure, Essigsäure, Oxalsäure, Weinsäure, Citronensäure, Ascorbinsäure, Sorbinsäure, Trimethylessigsäure, Benzoesäure, Salicylsäure, Bernsteinsäure, Maleinsäure, genannt.

Als Metallkomplex-Bildner sind vor allem Kationen der I. und II. oder der IV. bis VIII. Nebengruppe des Periodensystems der Elemente, insbesondere solche mit guter physiologischer Verträglichkeit, geeignet. Beispiele sind Kupfer, Zink, Mangan, Chrom, Eisen, Nickel, Kobalt, Molybdän.

Die vorstehenden Aufzählungen stellen keine Limitierungen dar. Der Fachmann kennt weitere physiologisch verträgliche Salze und Komplexbildner.

Verbindungen, worin R sek.Butyl darstellt, sollen hier und im folgenden gleichfalls zu den Milbemcyin-Derivaten gerechnet werden, obwohl sie nach der üblichen Systematik von Avermectin-Derivate abgeleitet sind. Avermectin-Aglykone(mit einer OH-Gruppe in 13α-Position) lassen sich jedoch gemäss US-PS 4,173,571 in Milbemycin-Homologe überführen.

In natürlich vorkommenden Milbemycinen (R = $CH_3$, $C_2H_5$ oder iso$C_3H_7$) ist die 13-Position anstelle der Vinylgruppe der erfindungsgemässen Verbindungen der Formel I ausschliesslich mit Wasserstoff besetzt, wie die nachstehende Formel XXX zeigt:

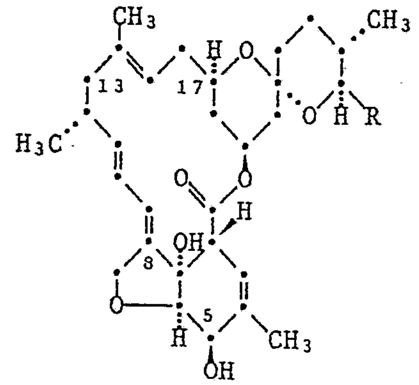

(XXX)

R = $CH_3$ Milbemycin $A_3$
R = $C_2H_5$ Milbemycin $A_4$
R = iso$C_3H_7$ Milbemycin D
R = sec.$C_4H_9$ 13-Deoxi-22,23,dihydro-C-076-Bla-aglycon.

Bei Avermectinen dagegen steht in der 13-Position ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten R = sek.$C_4H_9$ von den Milbemycinen. Durch Hydrolyse des Zucker-Restes der Avermectine gelangt man leicht zu den entsprechenden Avermectin-Aglykonen, die eine in Nachbarstellung zu einer C=C-Doppelbindung befindliche 13α-Hydroxy-Gruppe besitzen. Die Avermectin-Aglykone sind, vorstehend angegeben, in die Milbemycin-Homologen umwandelbar. Bei den Milbemycin-Derivaten der vorliegenden Anmeldung liegt die $\Delta^{22,23}$-Doppelbindung stets in hydrierter Form vor.

Verbindungen der Formel I, worin R für die Gruppe 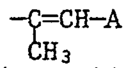 steht und A Methyl, Ethyl oder Isopropyl bedeutet, repräsentieren solche 23-Deoxy-Abkömmlinge der natürlich vorkommenden Antibiotika S541, die nunmehr in 13-Position eine Vinyl-Gruppierung enthalten.

Die Konstitution von natürlichen Antibiotika S541 ist aus der DE 35 32 794 bekannt und sieht wie folgt aus:

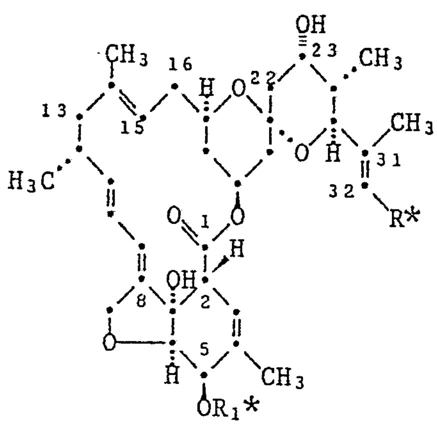

(Antibiotika S541)

| | | |
|---|---|---|
| Faktor A | $R^{*}=isoC_3H_7$ | $R_1{}^{*}=H$ |
| Faktor B | $R^{*}=CH_3$ | $R_1{}^{*}=CH_3$ |
| Faktor C | $R^{*}=CH_3$ | $R_1{}^{*}=H$ |
| Faktor D | $R^{*}=C_2H_5$ | $R_1{}^{*}=H$ |
| Faktor E | $R^{*}=C_2H_5$ | $R_1{}^{*}=CH_3$ |
| Faktor F | $R^{*}=isoC_3H_7$ | $R_1{}^{*}=CH_3$ |

Im Umfang der Formel I sind folgende interessante Gruppen zu nennen:

Gruppe Ia:
Verbindungen der Formel I, worin
R für Methyl, Ethyl, Isopropyl oder sek.Butyl oder für die Gruppe 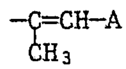 steht, wobei A Methyl, Ethyl oder Isopropyl bedeutet und

$R_1$ Wasserstoff darstellt.

Gruppe Ib:
Verbindungen der Formel I, worin
R für Methyl, Ethyl, Isopropyl oder sek.Butyl steht und
$R_1$ Wasserstoff darstellt.

Gruppe Ic:
Verbindungen der Formel I, worin
R für Methyl, Ethyl, Isopropyl oder sek.Butyl oder für die Gruppe 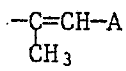 steht, wobei A Methyl, Ethyl oder Isopropyl bedeutet und

$R_1$ eine Silylgruppe darstellt.

Gruppe Id:
Verbindungen der Formel I, worin
R für Methyl, Ethyl, Isopropyl oder sek.Butyl steht und
$R_1$ eine Silylgruppe darstellt.

Gruppe Ie:
Verbindungen der Formel I, worin
R für Methyl, Ethyl, Isopropyl oder sek.Butyl oder für die Gruppe 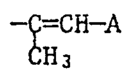 steht, wobei A Methyl, Ethyl oder Isopropyl bedeutet und

$R_1$ eine der Acylgruppen

a) 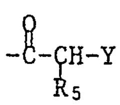 ,

b) 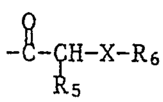 ,

c) 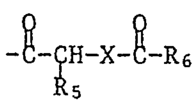 oder

d) 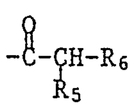

repräsentiert, wobei
X für Sauerstoff oder Schwefel steht;

Y für Halogen, Azido oder einen Sulfonsäurerest steht;
$R_5$ Wasserstoff, $C_1$-$C_4$-Alkyl oder Halogen bedeutet; und
$R_6$ für Wasserstoff, für unsubstituiertes oder durch Halogen, Hydroxy, $C_1$-$C_6$-Alkoxy oder $C_1$-$C_6$-Haloalkoxy substituiertes $C_1$-$C_{10}$-Alkyl, für einen unsubstituierten oder substituierten Rest ausgewählt aus der Reihe $C_3$-$C_{10}$-Cycloalkyl, $C_2$-$C_6$-Alkenyl und $C_3$-$C_6$-Alkinyl, wobei die Substituenten ausgewählt sind aus der Reihe Halogen, Hydroxy, $C_1$-$C_6$-Alkoxy und $C_1$-$C_6$-Alkanoyloxy, für unsubstituiertes oder durch Halogen, Cyano, $C_1$-$C_3$-Alkyl, $C_1$-$C_3$-Haloalkyl, $C_1$-$C_3$-Alkoxy, $C_1$-$C_3$-Haloalkoxy und/oder Nitro substituiertes Phenyl oder für einen unsubstituierten oder substituierten, ungesättigten oder gesättigten, fünf- oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

Innerhalb der Gruppe Ie sind folgende, die Gruppen If, Ig, Ih, Ii, Ik, Il, Im, In und Io bildenden Verbindungen der Formel I hervorzuheben.

Gruppe If:

Verbindungen der Formel I, worin
R für Methyl, Ethyl, Isopropyl oder sek.Butyl und
$R_1$ eine der Acylgruppen

a) 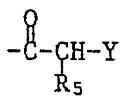 ,

b) 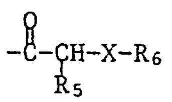 ,

c) 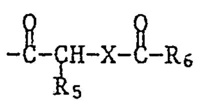 oder

d) 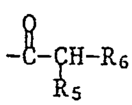

repräsentiert, wobei
X für Sauerstoff oder Schwefel steht;
Y für Halogen, Azido oder einen Sulfonsäurerest steht;
$R_5$ Wasserstoff, $C_1$-$C_4$-Alkyl oder Halogen bedeutet; und
$R_6$ für Wasserstoff, für unsubstituiertes oder durch Halogen, Hydroxy, $C_1$-$C_6$-Alkoxy oder $C_1$-$C_6$-Haloalkoxy substituiertes $C_1$-$C_{10}$-Alkyl, für einen unsubstituierten oder substituierten Rest ausgewählt aus der Reihe $C_3$-$C_{10}$-Cycloalkyl, $C_2$-$C_6$-Alkenyl und $C_3$-$C_6$Alkinyl, wobei die Substituenten ausgewählt sind aus der Reihe Halogen, Hydroxy, $C_1$-$C_6$-Alkoxy und $C_1$-$C_6$-Alkanoyloxy, für unsubstituiertes oder durch Halogen, Cyano, $C_1$-$C_3$-Alkyl, $C_1$-$C_3$-Haloalkyl, $C_1$-$C_3$-Alkoxy, $C_1$-$C_3$-Haloalkoxy und/oder Nitro substituiertes Phenyl oder für einen unsubstituierten oder substituierten, ungesättigten oder gesättigten, fünf- oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

Gruppe Ig:

Verbindungen der Formel I, worin
R für Methyl, Ethyl, Isopropyl oder sek.Butyl oder für die Gruppe 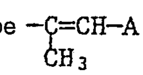 steht, wobei A Methyl, Ethyl oder Isopropyl bedeutet und

$R_1$ die Gruppe

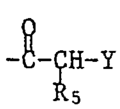

$R_5$ für Wasserstoff, Fluor oder Methyl steht; und

Y Chlor, Brom, Jod, Benzolsulfonyloxy, Paratosyloxy oder Mesyloxy bedeutet.

Gruppe Ih:
Verbindungen der Formel I, worin
R für Methyl, Ethyl, Isopropyl oder sek.Butyl steht;
$R_1$ die Gruppe

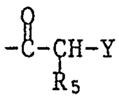

$R_5$ für Wasserstoff, Fluor oder Methyl steht; und
Y Chlor, Brom, Jod, Benzolsulfonyloxy, Paratosyloxy oder Mesyloxy bedeutet.

Gruppe Ii:
Verbindungen der Formel I, worin
R für Methyl, Ethyl, Isopropyl oder sek.-Butyl oder für die Gruppe 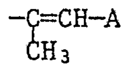 steht, wobei A Methyl, Ethyl oder Isopropyl bedeutet und

$R_1$ die Gruppe

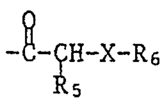

repräsentiert, wobei
X für Sauerstoff oder Schwefel steht;
$R_5$ Wasserstoff, Fluor oder Methyl bedeutet; und
$R_6$ für Wasserstoff, für unsubstituiertes oder durch Fluor, Chlor, Brom, $C_1$-$C_6$-Alkoxy, $C_3$-$C_6$-Alkoxyalkoxy, Thioalkoxy oder $C_1$-$C_3$-Haloalkoxy substituiertes $C_1$-$C_6$-Alkyl, für unsubstituiertes oder Fluor, Chlor, Brom, Methyl, Ethyl, Methoxy, Halomethoxy, Trifluormethyl und/oder Nitro substituiertes Phenyl oder Benzyl oder für einen unsubstituierten oder substituierten, ungesättigten oder gesättigten, fünf- oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht.

Gruppe Ik:
Verbindungen der Formel I, worin
R für Methyl, Ethyl, Isopropyl oder sek.-Butyl steht;
$R_1$ die Gruppe

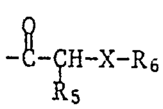

repräsentiert, wobei
X für Sauerstoff oder Schwefel steht;
$R_5$ Wasserstoff, Fluor oder Methyl bedeutet; und
$R_6$ für Wasserstoff, für unsubstituiertes oder durch Fluor, Chlor, Brom, $C_1$-$C_6$-Alkoxy, $C_3$-$C_6$-Alkoxyalkoxy, Thioalkoxy oder $C_1$-$C_3$-Haloalkoxy substituiertes $C_1$-$C_6$-Alkyl, für unsubstituiertes oder Fluor, Chlor, Brom, Methyl, Ethyl, Methoxy, Halomethoxy, Trifluormethyl und/oder Nitro substituiertes Phenyl oder Benzyl oder für einen unsubstituierten oder substituierten, ungesättigten oder gesättigten, fünf- oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht.

Gruppe Il:
Verbindungen der Formel I, worin
R für Methyl, Ethyl, Isopropyl oder sek.-Butyl oder für die Gruppe 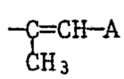 steht, wobei A Methyl, Ethyl oder Isopropyl bedeutet und

$R_1$ die Gruppe

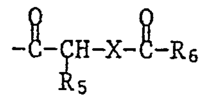

repräsentiert; wobei
X für Sauerstoff oder Schwefel steht;
$R_5$ Wasserstoff, Fluor oder Methyl bedeutet; und
$R_6$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf- oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht.

<u>Gruppe Im:</u>
Verbindungen der Formel I, worin
R für Methyl, Ethyl, Isopropyl oder sek.-Butyl steht;
$R_1$ die Gruppe

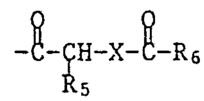

repräsentiert; wobei
X für Sauerstoff oder Schwefel steht;
$R_5$ Wasserstoff, Fluor oder Methyl bedeutet; und
$R_6$ für Wasserstoff, für unsubstituiertes oder durch Fluor, Chlor, Brom, Hydroxy, $C_1$-$C_3$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf- oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht.

<u>Gruppe In:</u>
Verbindungen der Formel I, worin
R für Methyl, Ethyl, Isopropyl oder sek.-Butyl oder für eine Gruppe 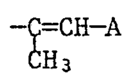 steht, wobei A Methyl, Ethyl oder Isopropyl bedeutet und

$R_1$ die Gruppe

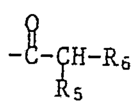

repräsentiert; wobei
$R_5$ für Wasserstoff, Fluor oder Methyl steht; und
$R_6$ für einen in 1-Position verknüpften, 5-gliedrigen Heteroaromaten mit 2 bis 3 Stickstoffatom steht, der unsubstituiert oder ein- bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

Gruppe Io:

Verbindungen der Formel I, worin

R für Methyl, Ethyl, Isopropyl oder sek.-Butyl steht;

$R_1$ die Gruppe

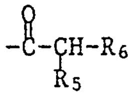

repräsentiert; wobei

$R_5$ für Wasserstoff, Fluor oder Methyl steht; und

$R_6$ für einen in 1-Position verknüpften, 5-gliedrigen Heteroaromaten mit 2 bis 3 Stickstoffatom steht, der unsubstituiert oder ein- bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

Bevorzugt werden auch folgende Substanzen:

Gruppe Ip:

Verbindungen der Formel I, worin

R für Methyl, Ethyl, Isopropyl oder sek.-Butyl oder für eine Gruppe 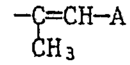 steht, wobei A Methyl, Ethyl oder Isopropyl bedeutet und

$R_1$ eine der Acylgruppen

a) 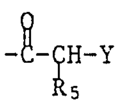 ,

b) 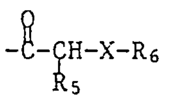 ,

c) 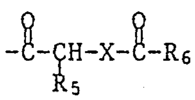 oder

d) 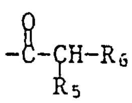

repräsentiert, wobei

X für Sauerstoff oder Schwefel steht;

Y Halogen bedeutet;

$R_5$ Wasserstoff, Halogen oder Methyl repräsentiert; und

$R_6$ für Wasserstoff, unsubstituiertes oder durch Halogen oder $C_1$-$C_4$-Alkoxy substituiertes $C_1$-$C_4$-Alkyl steht, für Cyclopropyl, für Cyclopentyl, für Cyclohexyl, für unsubstituiertes oder durch Fluor, Chlor, Brom, Methoxy, Trifluormethyl, Methyl und/oder Nitro substituiertes Phenyl, für 4H-2,3-Dihydropyran-2-yl oder für einen in 1-Position verknüpften, 5-gliedrigen Heteroaromaten mit 2 bis 3 Stickstoffatom steht, der unsubstituiert oder ein-bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

Gruppe Iq:

Verbindungen der Formel I, worin

R für Methyl, Ethyl, Isopropyl oder sek.-Butyl steht;

$R_1$ eine der Acylgruppen

a) 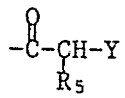 ,

b) 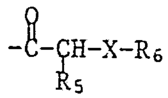 ,

c) 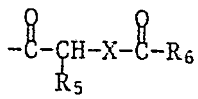 oder

d) 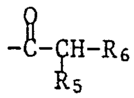

repräsentiert, wobei
X für Sauerstoff oder Schwefel steht;
Y Halogen bedeutet;
$R_5$ Wasserstoff, Halogen oder Methyl repräsentiert; und
$R_6$ für Wasserstoff, unsubstituiertes oder durch Halogen oder $C_1$-$C_4$-Alkoxy substituiertes $C_1$-$C_4$-Alkyl steht, für Cyclopropyl, für Cyclopentyl, für Cyclohexyl, für unsubstituiertes oder durch Fluor, Chlor, Brom, Methoxy, Trifluormethyl, Methyl und/oder Nitro substituiertes Phenyl, für 4H-2,3-Dihydropyran-2-yl oder für einen in 1-Position verknüpften, 5-gliedrigen Heteroaromaten mit 2 bis 3 Stickstoffatom steht, der unsubstituiert oder ein-bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

Besonders bevorzugte Einzelsubstanzen der Formel I sind beispielsweise:
-13β-Vinyl-milbemycin D,
-13β-Vinyl-milbemycin $A_4$,
-13β-Vinyl-milbemycin $A_3$,
-13α-Vinyl-milbemycin D,
-13α-Vinyl-milbemycin $A_4$,
-13α-Vinyl-milbemycin $A_3$,

5-O-tert.-Butyldimethylsilyl-13β-vinyl-milbemycin D,
5-O-tert.-Butyldimethylsilyl-13β-vinyl-milbemycin $A_4$,
5-O-tert.-Butyldimethylsilyl-13β-vinyl-milbemycin $A_3$,
5-O-tert.-Butyldimethylsilyl-13α-vinyl-milbemycin D,
5-O-tert.-Butyldimethylsilyl-13α-vinyl-milbemycin $A_4$,
5-O-tert.-Butyldimethylsilyl-13α-vinyl-milbemycin $A_3$,

5-O-Chloracetyl-13β-vinyl-milbemycin D,
5-O-Chloracetyl-13β-vinyl-milbemycin $A_4$,
5-O-Chloracetyl-13β-vinyl-milbemycin $A_3$,
5-O-Chloracetyl-13α-vinyl-milbemycin D,
5-O-Chloracetyl-13α-vinyl-milbemycin $A_4$,
5-O-Chloracetyl-13α-vinyl-milbemycin $A_3$,

5-O-Acetoxyacetyl-13β-vinyl-milbemycin D,
5-O-Acetoxyacetyl-13β-vinyl-milbemycin $A_4$,
5-O-Acetoxyacetyl-13β-vinyl-milbemycin $A_3$,
5-O-Acetoxyacetyl-13α-vinyl-milbemycin D,
5-O-Acetoxyacetyl-13α-vinyl-milbemycin $A_4$,
5-O-Acetoxyacetyl-13α-vinyl-milbemycin $A_3$,

5-O-Methoxyacetyl-13β-vinyl-milbemycin D,
5-O-Methoxyacetyl-13β-vinyl-milbemycin $A_4$,
5-O-Methoxyacetyl-13β-vinyl-milbemycin $A_3$,
5-O-Methoxyacetyl-13α-vinyl-milbemycin D,
5-O-Methoxyacetyl-13α-vinyl-milbemycin $A_4$,
5-O-Methoxyacetyl-13α-vinyl-milbemycin $A_3$,

5-O-[3-Chlorobenzoyloxy]acetyl-13β-vinyl-milbemycin D,
5-O-[3-Chlorobenzoyloxy]acetyl-13β-vinyl-milbemycin $A_4$,
5-O-[3-Chlorobenzoyloxy]acetyl-13β-vinyl-milbemycin $A_3$,
5-O-[3-Chlorobenzoyloxy]acetyl-13α-vinyl-milbemycin D,
5-O-[3-Chlorobenzoyloxy]acetyl-13α-vinyl-milbemycin $A_4$,
5-O-[3-Chlorobenzoyloxy]acetyl-13α-vinyl-milbemycin $A_3$,

5-O-[3,4-Dihydro-2H-pyran-2-yl]carboxyacetyl-13β-vinyl-milbemycin D,
5-O-[3,4-Dihydro-2H-pyran-2-yl]carboxyacetyl-13β-vinyl-milbemycin $A_4$,
5-O-[3,4-Dihydro-2H-pyran-2-yl]carboxyacetyl-13β-vinyl-milbemycin $A_3$,
5-O-[3,4-Dihydro-2H-pyran-2-yl]carboxyacetyl-13α-vinyl-milbemycin D,
5-O-[3,4-Dihydro-2H-pyran-2-yl]carboxyacetyl-13α-vinyl-milbemycin $A_4$,
5-O-[3,4-Dihydro-2H-pyran-2-yl]carboxyacetyl-13α-vinyl-milbemycin $A_3$,

5-O-[1,2,4-Triazol-1-yl]acetyl-13β-vinyl-milbemycin D,
5-O-[1,2,4-Triazol-1-yl]acetyl-13β-vinyl-milbemycin $A_4$,
5-O-[1,2,4-Triazol-1-yl]acetyl-13β-vinyl-milbemycin $A_3$,
5-O-[1,2,4-Triazol-1-yl]acetyl-13α-vinyl-milbemycin D,
5-O-[1,2,4-Triazol-1-yl]acetyl-13α-vinyl-milbemycin $A_4$,
5-O-[1,2,4-Triazol-1-yl]acetyl-13α-vinyl-milbemycin $A_3$.

Die vorliegende Erfindung betrifft auch Verfahren, die es erlauben, in der 13-Position von Milbemycin-, 13-Deoxi-22,23-dihydro-Avermectinaglykon-Derivaten oder von Derivaten der natürlichen Antibiotika S541 eine α- und/oder β-Vinylgruppe gezielt einzuführen und Verfahren, die es erlauben in der 5-O-Position von 13-Vinyl-Milbemycin-, 13-Deoxi-13-Vinyl-22,23-dihydro-Avermectin-aglykon-Derivaten oder 13-Vinyl-23-deoxy-Derivaten der natürlichen Antibiotika S541 eine 5-O-Acylgruppe gezielt einzuführen um damit zu den hochwirksamen neuen Parasitiziden der Formel I zu gelangen.

Zur Herstellung von Verbindungen der Formel I, welche an der 5-O-Position eine Acylgruppe tragen, wird erfindungsgemäss folgendes Verfahren durchgeführt, in dem man nämlich ein 13-Vinyl-Milbemycin-Derivat der Formel I, wobei $R_1$ = Wasserstoff bedeutet, mit einer geeigneten Säure z.B. einer Säure der Formel II, einem ihrer Säurehalogenide oder ihrem Säureanhydrid an der 5-OH-Gruppe verestert, wobei der Rest $-\overset{O}{\overset{\|}{C}}R_7$ für eine in den Gruppen Ie bis Io definierte Acylgruppe steht.

$$HO-\overset{O}{\overset{\|}{C}}R_7 \quad (II)$$

Bevorzugte Säurehalogenide der Säuren II sind deren Chloride und Bromide.

Im Falle der Säurehalogenide und Säureanhydride von II erfolgt die Umsetzung vorzugsweise in einem inerten, hydroxylgruppenfreien Lösungsmittel in Anwesenheit einer organischen Base, wie z.B. Pyridin, 4-Dimethylaminopyridin, Lutidin, Collidin, Trialkylamin, N-Dialkylanilin, oder bicyclische, nicht nucleophile Basen wie z.B. 1,4-Diazabicyclo[2.2.2]octan (DABCO), 1,5-Diazabicyclo[4.3.0]non-5-en (DBN) oder 1,8-Diazabicylo[5.4.0]undec-7-en (1,5-5) (DBU). Die Reaktion wird im allgemeinen bei Temperaturen von -30° bis +70°C, vorzugsweise von -10° bis +50°C durchgeführt. Man arbeitet dabei zweckmässigerweise in Gegenwart eines reaktionsinerten Lösungsmittels oder Lösungsmittelgemisches. Es eignen sich hierfür z.B. aliphatische und aromatische Kohlenwasserstoffe wie Benzol, Toluol, Xylole, Petrolether, Hexan; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; Ester wie Ethylacetat (Essigsäureethylester), Propylacetat oder Butylacetat; Ketone wie Aceton, Diethylketon, Methylethylketon; Verbindungen wie Dimethylsulfoxid (DMSO), Dimethylformamid (DMF) und Gemische solcher Lösungsmittel untereinander. Man kann die Reaktion aber auch im Ueberschuss einer der oben genannten Basen durchführen.

Geht man von der Säure der Formel II selbst aus, so wird die Veresterungsreaktion I ($R_1$ = Wasserstoff) mit II vorteilhafterweise in Gegenwart für Veresterungen üblicher, wasserabspaltenden Reagenzien durchgeführt, wie z.B. in Anwesenheit eines Carbodiimids [Dicyclohexylcarbodiimid (DCC)] oder eines 1-Alkyl-2-halogenpyri diniumsalzes [1-Methyl-2-chlorpyridiniumjodid]. Diese Reaktion wird vorzugsweise in einem der obengenannten, reaktionsinerten Lösungsmittel und bei Temperaturen von -30° bis +70°C, vorzugsweise -10° bis +50°C durchgeführt. Man arbeitet bevorzugt in Gegenwart einer Base wie z.B. in Gegenwart eines organischen Amins z.B. eines Trialkylamins (Trimethylamin, Triethylamin, Tripropylamin, Diisopropylethylamin usw.), eines Pyridins (Pyridin selbst, 4-Dimethylaminopyridin, 4-Pyrrolidylaminopyridin usw.), eines Morpholins (N-Methylmorpholin), eines N,N-Dialkylanilins (N,N-Dimethylanilin, N-Methyl-N-ethylanilin) usw.

Verbindungen der Formel I wobei $R_1$ eine Acylgruppe darstellt, können aber auch durch geeignete Reaktionen aus anderen Verbindungen der Formel I hergestellt werden.

Beispielsweise lässt sich eine Verbindung mit $R_1$ = $-CO-CH_2Cl$ durch Umsetzung mit Natriumacetat in DMF bei 80°C in eine Verbindung mit $R_1$ = $-CO-CH_2-OCOCH_3$ umwandeln. Diese wiederum lässt sich beispielsweise auch aus einer Verbindung mit $R_1$ = $-CO-CH_2-OH$ durch Veresterung mit Acetylchlorid oder Essigsäureanhydrid herstellen. Derartige Umwandlungen von Verbindungen der Formel I in andere Vertreter

der Formel I werden als zum Hauptverfahren gehörig betrachtet.

Das beschriebene Herstellungsverfahren ist einschliesslich aller Teilstufen essentieller Bestandteil der vorliegenden Erfindung.

Die Säuren der Formel II, ihre Halogenide und Anhydride sind grösstenteils bekannt oder lassen sich analog zu den bekannten Vertretern herstellen.

Zur Herstellung von Verbindungen der Formel I, wobei $R_1$ für Wasserstoff oder eine Silylgruppe steht, wird erfindungsgemäss folgendes Verfahren durchgeführt, indem man nämlich eine Verbindung der Formel III,

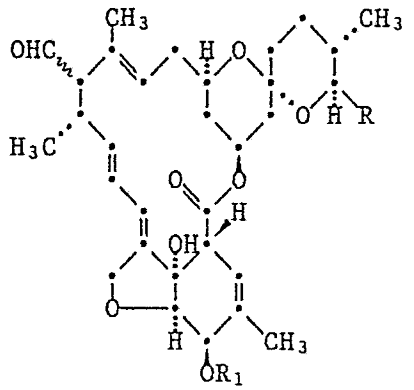

(III)

worin R die unter Formel I angegebenen Bedeutungen hat, und $R_1$ für Wasserstoff oder eine Schutzgruppe, bevorzugt eine Silylgruppe steht, mit einem zur Freisetzung des Titankomplexes der Formel IV

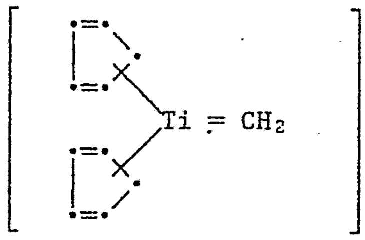

(IV)

befähigten Reagenz umsetzt.

Geeignete Reagenzien dieser Art sind aus der Literatur [R.H. Grubbs et al. Pure & Appl. Chem. 55, pp 1733-1738 (1983)] bekannt oder lassen sich analog zu den dort genannten Vertretern herstellen.

Besonders geeignet ist die Verbindung der Formel V

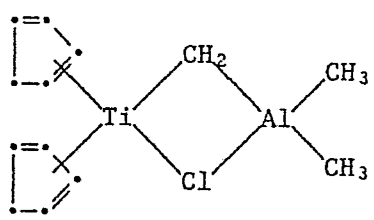

(V)

Im allgemeinen wird die Reaktion so durchgeführt, dass die Verbindung der Formel IV in situ im Reaktionsmedium aus der Verbindung der Formel V gebildet wird. Man arbeitet üblicherweise bei Temperaturen von -80° bis +30°C, zweckmässigerweise in Gegenwart eines reaktionsinerten Lösungsmittels oder Lösungsmittelgemisches. Es eignen sich hierfür z.B. aliphatische und aromatische Kohlenwasserstoffe wie Benzol, Toluol, Xylole, Petrolether, Hexan und etherartige Verbindungen wie Dialkylether (Diethylether, Diisopropylether, tert.-Butylmethylether usw.), Dioxan, Tetrahydrofuran usw. und Gemische dieser untereinander.

Man arbeitet häufig in Anwesenheit einer katalytischen Menge einer organischen Base, wie z.B. Pyridin, 4-Dimethylaminopyridin, Lutiden, N-Dialkylanilin (Dimethylanilin) oder eines Trialkylamins (Triethylamin).

Verbindungen der Formel I, wobei $R_1$ für Wasserstoff oder eine Silylgruppe steht, können aber auch durch Wittig-Reaktion analog bekannter Vorschriften hergestellt werden.

Verbindungen der Formel III werden erfindungsgemäss hergestellt, in dem man eine Verbindung der Formel

VI, worin R und $R_1$ die unter Formel I angegebenen Bedeutungen haben, in einem inerten Lösungsmittel mit einer Lewis- oder einer Brönsted-Säure behandelt. Die Reaktion wird im allgemeinen im Temperaturbereich von -50°C bis +50°C durchgeführt.

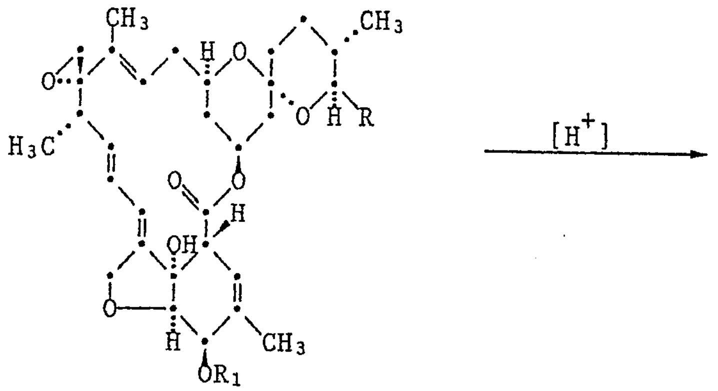


(VI) (III)

Als inerte Lösungsmittel eignen sich z.B. aliphatische und aromatische Kohlenwasserstoffe wie Benzol, Toluol, Xylole, Petrolether, Hexan; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; und Gemische solcher Lösungsmittel untereinander.

Als Lewis-Säuren seien speziell erwähnt Bortrifluor-äthylätherat, Titantetrachlorid, Lithiumbromid, Lithiumperchlorat, Zinkchlorid und besonders Magnesiumbromid. Als Brönsted-Säuren kommen z.B. HCl in Aether, HF in Acetonitril, Oxalsäure und besonders Sulfonsäuren, wie Methansulfonsäure, p-Toluolsulfonsäure und Campher-10-sulfonsäure in Frage. Diese Aufzählung hat nur beispielhaften Charakter und stellt keine Limitierung dar.

Verbindungen der Formel III werden dabei als Gemisch von 13β- und 13α-Formyl-Milbemycin-Derivaten erhalten. Das Verhältnis lässt sich durch die Wahl der Reaktionsparameter (z.B. Temperatur, Lösungsmittel, Reagenz) beeinflussen.

Die Verbindungen der Formeln III und VI stellen auf Grund ihrer Struktur und ihres reaktiven Zentrums in der 13-Position wertvolle Zwischenprodukte zur Synthese der 13-Vinyl-Milbemycine der Formel I dar und sind daher Bestandteile der vorliegenden Erfindung.

Verbindungen der Formel VI werden erfindungsgemäss hergestellt, indem man eine Verbindung der Formel VIII, worin R und $R_1$ die unter Formel I angegebenen Bedeutungen haben, in einem inerten Lösungsmittel im Temperaturbereich von -50°C bis +80°C mit einem Dialkylsulfoniummethylid der Formel VIII umsetzt,

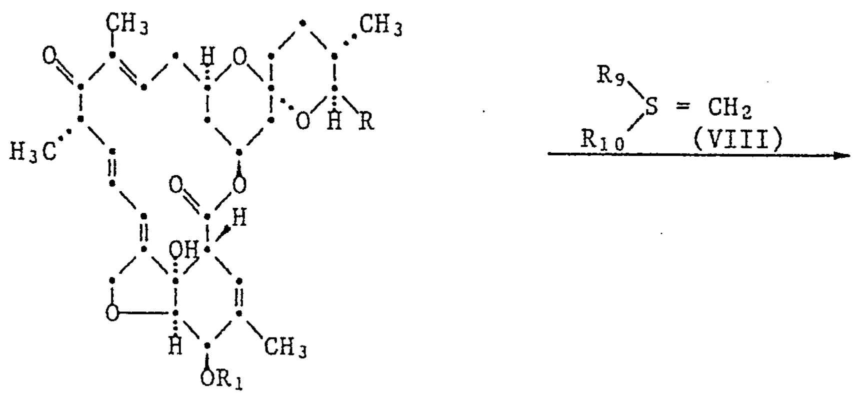

(VIII) (VI)

(VII)

wobei $R_9$ und $R_{10}$ unabhängig voneinander eine $C_1$-$C_{15}$-Alkylgruppe bedeuten.

Verbindungen der Formel VIII werden dabei meist in situ aus Verbindungen der Formel IX durch Behandlung mit einer starken Base wie z.B. Butyllithium, Methyllithium, Kaliumhydrid, Natriumhydrid, Natriumhydroxid (NaOH) und Kaliumhydroxid (KOH) hergestellt.

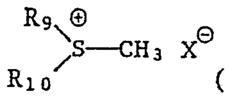 (IX)

$X^{\ominus}$ z.B. $Cl^{\ominus}$, $Br^{\ominus}$, $J^{\ominus}$, $CH_3SO_4^{\ominus}$

Als Lösungsmittel eignen sich z.B. aliphatische und aromatische Kohlenwasserstoffe wie Benzol, Toluol, Xylole, Petrolether, Hexan; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Verbindungen wie Dimethylsulfoxid (DMSO), Dimethylformamid (DMF) und Gemische solcher Lösungsmittel untereinander. Falls $R_9$ und/oder $R_{10}$ für eine $C_4$-$C_{15}$-Alkylgruppe steht kann aber auch im 2-Phasengemisch eines dieser Lösungsmittels bzw. eines Gemisches solcher Lösungsmittel und einer wässriger Base wie z.B. NaOH oder KOH gearbeitet werden.

Verbindungen der Formel VII, worin R für Methyl, Ethyl, Isopropyl oder sek.Butyl steht und $R_1$ die unter Formel I angegebene Bedeutung hat, sind bekannt aus EP 0180539.

Verbindungen der Formel VII, worin R für die Gruppe 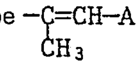 steht und A Methyl, Ethyl oder Isopropyl bedeutet und $R_1$ Wasserstoff, eine Silyl- oder eine Acylgruppe bedeutet, lassen sich analog zu an sich bekannten Verfahren aus den natürlich vorkommenden Antibiotika S541 herstellen, die aus der DE 35 32 794 bekannt und durch die folgende chemische Struktur charakterisiert sind:

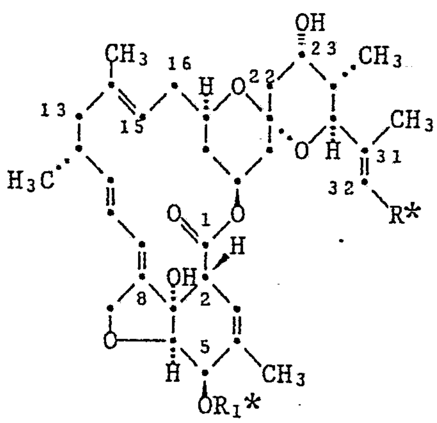

(A)

(Antibiotika S541)

| | | |
|---|---|---|
| Faktor A | $R^{*}=isoC_3H_7$ | $R_1^{*}=H$ |
| Faktor B | $R^{*}=CH_3$ | $R_1^{*}=CH_3$ |
| Faktor C | $R^{*}=CH_3$ | $R_1^{*}=H$ |
| Faktor D | $R^{*}=C_2H_5$ | $R_1^{*}=H$ |
| Faktor E | $R^{*}=C_2H_5$ | $R_1^{*}=CH_3$ |
| Faktor F | $R^{*}=isoC_3H_7$ | $R_1^{*}=CH_3$ |

Je nach Faktor werden im folgenden zur Vereinfachung der Benennung die Abkömmlinge von Antibiotikum S541 als Derivate von S541A, S541B, S541C, S541D, S541E oder S541F klassifiziert.

Die in 23-Position befindliche Hydroxygruppe in den Antibiotika S541 lässt sich analog zu der in US-PS 4,328,335 bekanntgewordenen Methode entfernen und die Antibiotika S541 lassen sich so in die entsprechenden 23-Deoxi-Derivate überführen. Dabei müssen diejenigen Verbindungen mit einer freien 5-OH-Gruppe ($R_1^{*}=H$) zunächst selektiv geschützt werden durch Reaktion mit einem der zuvor genannten Silylierungsreagenzien Y-Si($R_2$)($R_3$)($R_4$) bzw. mit tert.-Butyldimethylsilyloxiacetylchlorid. Die Umsetzung dieser geschützten Verbindungen, in denen $R_1^{*}$ durch Si($R_2$)($R_3$)($R_4$) bzw. $C(=O)CH_2OSi(CH_3)_2t\text{-}C_4H_9$ ersetzt und das 23-C-Atom durch OH substituiert ist, mit p-Methylphenyl-chlorthionoformiat ergibt Derivate

der Antibiotika S541, welche in Position 23 durch p-$CH_3$-$C_6H_4$-O-C(=S)-O- substituiert sind. Diese 23-O-(4-Methyl-phenoxi)-thiocarbonyl-derivate von Antibiotika S541 werden dann als Ausgangsmaterialien für die Reduktion mit Tributylzinnhydrid in Toluol in Gegenwart von Azobisisobutyronitril bei 80-120°C zu den entsprechenden 23-Deoxi-Derivaten (Position 23 unsubstituiert) eingesetzt.

Die 13-Oxo-Verbindungen von den 23-Deoxi-Derivaten der natürlichen Antibiotika S541 können entsprechend dem in EP 0180539 beschriebenen Verfahren hergestellt werden.

In einem ersten Verfahrensschritt werden dabei die 23-Deoxi-Derivate der natürlichen Antibiotika S541 durch Epoxidierung mit einer Persäure wie z.B. m-Chlorperbenzoesäure im Temperaturbereich von -10°C bis Raumtemperatur in einem inerten Lösungsmittel wie Dichlormethan in die entsprechenden 14,15-Epoxi-Verbindungen übergeführt. Dabei kann $R_1$* Wasserstoff oder eine Schutzgruppe bedeuten wie z.B. eine Silylgruppe $Si(R_2)(R_3)(R_4)$. Die $\Delta^{13,14}$-15-Hydroxi-Derivate lassen sich aus den 14,15-Epoxiden mit Hilfe des Komplex-Reagenzes $[NH_3]_m/[Al(Ethyl)_3]_n$, worin m und n unabhängig voneinander die Zahl 1 oder 2 oder einen Zahlenwert zwischen 1 und 2 darstellen, herstellen. Man arbeitet dabei in inerten trockenen Lösungsmitteln im Temperaturbereich von -30°C bis +100°C, vorzugsweise von 0°C bis +70°C. Als inerte Lösungsmittel kommen vorzugsweise aliphatische und aromatische Kohlenwasserstoffe wie Benzol, Toluol, Xylol, Petrolether; Ether wie Diethylether, tert.-Butylmethylether, Tetrahydrofuran, Dioxan und Anisol in Frage.

Die nachfolgenden Umsetzungen werden jeweils mit Verbindungen, deren 5-OH-Gruppe ($R_1$* = H) selektiv geschützt ist, durchgeführt. Besonders geeignete Schutzgruppen sind beispielsweise die zuvor genannten Silylgruppen -$Si(R_2)(R_3)(R_4)$.

Die 13-Hydroxi-Derivate werden durch Reaktion der $\Delta^{13,14}$-15-Hydroxi-Verbindungen mit Chromat-, Halochromat-, oder Dichromat-Ionen, insbesondere mit Pyridiniumdichromat [=$(Pyr)_2^+Cr_2O_7$] oder mit Pyridiniumchlorochromat [=$(Pyr)^+ClCrO_3$] hergestellt.

Es werden inerte wasserfreie, vorzugsweise polare Lösungsmittel, z.B. Dimethylformamid (=DMF) verwendet. Die Reaktion wird bei Temperaturen von -10°C bis +60°C, bevorzugt +10°C bis +40°C, durchgeführt.

Es werden bei dieser Reaktion neben den entsprechenden 13β-Hydroxi-Verbindungen auch die 13-Oxo-Derivate gebildet. Die beiden so erhaltenen Reaktionsprodukte lassen sich durch übliche Trennungsmethoden, z.B. durch fraktionierte Kristallisation oder durch Chromatographie, voneinander trennen. Unter Chromatographie werden Säulen-, Dickschicht- oder Dünnschicht-Chromatographie an mineralischen Trägermaterialien wie Silicagel oder an organischen Austauscherharzen verstanden.

Die 13-Oxo-Derivate von den 23-Deoxi-Verbindungen der natürlichen Antibiotika S541 lassen sich gezielt aus den entsprechenden 13β-Hydroxi-Derivaten durch Oxidation mit Dimethylsulfoxid (DMSO)/Oxalylchlorid herstellen. Die Reaktion wird vorzugsweise bei Temperaturen von -80°C bis Raumtemperatur durchgeführt. Als Lösungsmittel kommen DMSO selbst, sowie aromatische Kohlenwasserstoffe wie Benzol, Toluol, Xylole und chlorierte Kohlenwasserstoffe, wie Methylenchlorid in Frage. Man arbeitet vorzugsweise unter Zugabe einer Base wie z.B. Triethylamin.

Durch Silylierung der 5-OH-Gruppe werden alle jene Derivate der Formeln III, VI und VII hergestellt, bei denen $R_1$ eine andere Bedeutung als Wasserstoff ($R_1$ = OH-Schutzgruppe) hat. Zur Silylierung verwendet man zweckmässigerweise ein Silan der Formel Y-$Si(R_2)(R_3)(R_4)$, wobei $R_2$, $R_3$ und $R_4$ vorzugsweise unabhängig voneinander für $C_1$-$C_6$-Alkyl, Benzyl oder Phenyl stehen und beispielsweise eine der Gruppen Trimethylsilyl, tris(tert.Butyl)silyl, Thexyldimethylsilyl, Diphenyl-tert.butylsilyl, bis(Isopropyl)methylsilyl, Triphenylsilyl usw. und insbesondere tert.Butyl-dimethylsilyl bildet. Die 5-OH-Gruppe kann auch als Benzylether oder Methoxiethoximethylether vorliegen. Zu den Silylabgangsgruppen Y zählen beispielsweise Bromid, Chlorid, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0° bis +80°C, bevorzugt bei +10° bis +40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung der Silylreste in der 5-Position geschieht durch selektive milde Hydrolyse mit z.B. Arylsulfonsäure in alkoholischer Lösung, HF in Acetonitril, $HF_x$•Pyridin in Tetrahydrofuran oder nach einer anderen dem Fachmann geläufigen Methode.

Verbindungen der Formeln I und III können als Gemische der entsprechenden 13β- und 13α-Epimeren vorliegen. Die reinen Diastereomeren lassen sich daraus durch eine physikalische Trennoperation rein darstellen. Als physikalische Trennoperationen eignen sich z.B. Säulenchromatographie, Flash-Chromatographie, Dickschichtchromatographie, HPLC und Kristallisation.

Die meisten Reaktionen werden vorteilhaft unter Schutzgas wie z.B. Stickstoff oder Argon durchgeführt.

Das beschriebene Verfahren zur Herstellung der Verbindungen der Formel I ist in allen Teilschritten ein Bestandteil vorliegender Erfindung.

Ein weiterer Gegenstand vorliegender Erfindung betrifft Schädlingsbekämpfungsmittel gegen Ekto- und Endoparasiten sowie Schadinsekten, die neben üblichen Trägerstoffen und/oder Verteilungsmitteln als mindestens einen Wirkstoff eine Verbindung der Formel I enthalten.

Die Verbindungen der Formel I eignen sind ausgezeichnet zur Bekämpfung von Schädlingen an Tieren und Pflanzen in allen ihren Entwicklungsstadien , darunter tierparasitären Ekto-Parasiten. Zu letzteren zählen unter

der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Besonders aber sind die Verbindungen gegen Helminthen in allen Entwicklungsstadien wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostonum, Oesophagostomum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Gattungen Nematodirus, Cooperia und Oesophagostomum greifen den Intestinaltrakt des Wirtstiers an, während andere der Gattungen Haemonchus und Ostertagia im Magen und solche der Gattung Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Gattungen Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Gattungen Wuchereria, Brugia, Onchocerca und Loa aus der Familie der Filariidae, die im Blut, im Gewebe und verschiedenen Organen vorkommen, ferner gegen Dracunculus und Parasiten der Gattungen Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet. Falls die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

Ueber geschlossenen Kultur-Anbauflächen, in Pferchen, Stallungen oder sonstigen Räumen werden die Verbindungen der Formel I in Aufwandmengen von 10 g bis 1000 g pro Hektar angewendet.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben: "1986 International McCutcheon's Emulsifiers and Detergents" The Manufacturing Confectioner Publishing Co., Glen Rock, New Jersey, USA.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

## Herstellung von Zwischenprodukten

### A1. Herstellung von 5-O-tert.-Butyldimethylsilyl-13,13-methylenoxymilbemycin $A_4$

Zu einer Suspension von 1,020 g fein pulverisiertem Trimethylsulfoniumjodid in 5 ml Tetrahydrofuran (THF) wurden bei 0°C innert 10 Min. 2,50 ml einer 1,6 M Lösung von n-Butyllithium in Hexan gegeben. Nach 30 Min. Rühren bei 0°C wurde das Reaktionsgemisch mit einer Lösung von 1,342 g 5-O-tert.-Butyldimethylsilyl-13-oxo-milbemycin $A_4$ in 5 ml THF innert 5 Min. versetzt. Nach 90 Min. Rühren bei 0°C wurde das Reaktionsgemisch auf 50 ml $H_2O$ gegossen und 3x mit 100 ml Aether extrahiert. Die organischen Phasen wurden mit 50 ml ges. NaCl-Lösung gewaschen, mit $MgSO_4$ getrocknet und eingedampft. Chromatographie des Rohprodukts an Kieselgel mit Hexan/Essigsäureäthyläther 10:1 ergab neben 201 mg Edukt 665 mg Produkt.

MS.: (m/e): 684 ($M^+$, $C_{39}H_{60}O_8Si$)
$^1H$-NMR (300 MHz, $CDCl_3$):
2,37 ppm (d, J = 6) 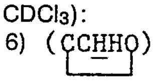
2,84 ppm (d, J = 6) 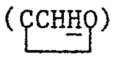
3,05 ppm (dt, $J_d$ = 2,5, $J_t$ = 9) ($C_{25}H$)
4,43 ppm (bs, $W_{1/2}$ = 11) ($C_5H$)

A2. Herstellung von 13,13-Methylenoxy-milbemycin $A_4$

Eine Lösung von 50 mg 5-O-tert.-Butyldimethylsilyl-13,13-methylenoxy-milbemycin $A_4$ in 1,0 ml einer $HF_x$•Pyridin/THF-Lösung (hergestellt aus 6,5 ml $HF_x$•Pyridin, 15,7 ml Pyridin und 50 ml THF) wurde 18 Std. bei Raumtemperatur gerührt. Das Reaktionsgemisch wurde dann auf 50 ml ges. $NaHCO_3$-Lösung gegossen und mit 100 ml Diäthyläther extrahiert. Die organische Phase wurde mit 50 ml ges. NaCl-Lösung gewaschen, mit $MgSO_4$ getrocknet und eingedampft. Nach Chromatographie des Rohprodukts an Kieselgel mit Hexan/Essigsäureäthylester 2:1 erhielt man 39 mg Produkt.
MS: (m/e): 570 ($M^+$, $C_{33}H_{46}O_8$)
$^1H$-NMR (300 MHz, $CDCl_3$):
2,37 ppm (d, J = 6) 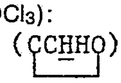
2,84 ppm (d, J = 6) 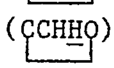
3,04 ppm (dt, $J_d$ = 2,5, $J_t$ = 9) ($C_{25}H$)
4,28 ppm (t, J = 7,5) ($C_5H$)

Analog zu den Beispielen A1 und A2 werden die nachfolgenden Verbindungen hergestellt.
5-O-tert.-Butyldimethylsilyl-13,13-methylenoxy-milbemycin $A_3$,
5-O-tert.-Butyldimethylsilyl-13,13-methylenoxy-milbemycin D,
13,13-Methylenoxy-milbemycin $A_3$ und 13,13-Methylenoxy-milbemycin D

B1. Herstellung von 5-O-tert.-Butyldimethylsilyl-13-formyl-milbemycin $A_4$

Eine Lösung von 465 mg 5-O-tert.-Butyldimethylsilyl-13,13-methylenoxy-milbemycin $A_4$ in 7 ml Methylenchlorid wurde bei 0°C mit 165 mg (±)-Campher-10-sulfonsäure versetzt und anschliessend 1 Std. bei 0°C gerührt. Das Reaktionsgemisch wurde dann auf 50 ml ges. $NaHCO_3$-Lösung gegossen und 3x mit 100 ml Diäthyläther extrahiert. Die organischen Phasen wurden mit 50 ml ges. NaCl-Lösung gewaschen, mit $MgSO_4$ getrocknet und eingedampft. Nach Chromatographie des Rohprodukts an Kieselgel mit Hexan/Essigsäureäthylester 6:1 konnten 371 mg Produkt als Epimerengemisch an C(13) (13β:13α ca. 2:1) erhalten werden.
MS: (m/e): 684 ($M^+$, $C_{39}H_{60}O_8Si$)

Mit Hilfe von physikalischen Trennoperationen gelang es die beiden Diastereomeren rein darzustellen:

5-O-tert.-Butyldimethylsilyl-13β-formyl-milbemycin $A_4$ $^1H$-NMR (300 MHz, $CDCl_3$):
2,82 ppm (m) ($C_{12}H$)
2,90 ppm (dd, $J_1$ = 1, $J_2$ = 10,5) ($C_{13}H$)
3,05 ppm (dt, $J_d$ = 2,5, $J_t$ = 9) ($C_{25}H$)
4,42 ppm (bs, $W_{1/2}$ = 11) ($C_5H$)
9,60 ppm (d, J = 1) (CHO)

5-O-tert.-Butyldimethylsilyl-13α-formyl-milbemycin $A_4$ $^1H$-NMR (300 MHz, $CDCl_3$):
2,46 ppm (m) ($C_{12}H$)
2,99 ppm (bs, $W_{1/2}$ = 9) ($C_{13}H$)
3,03 ppm (dt, $J_d$ = 2,5, $J_t$ = 9) ($C_{25}H$)
4,42 ppm (bs, $W_{1/2}$ = 11) ($C_2H$)
9,76 ppm (bs, $W_{1/2}$ = 4) (CHO)

B2. Herstellung von 13-Formyl-milbemycin $A_4$

a) Eine Lösung von 105 mg 5-O-tert.-Butyldimethylsilyl-13,13-methylenoxy-milbemycin $A_4$ in 0,5 ml Dichlormethan wurde mit 1,5 ml einer 5% Lösung von 40 % wässrigem HF in Acetonitril versetzt und anschliessend 1 Std. bei Raumtemperatur gerührt. Das Reaktionsgemisch wurde dann auf 50 ml ges. $NaHCO_3$-Lösung gegossen und mit 100 ml Diäthyläther extrahiert. Die organische Phase wurde mit 50 ml ges. NaCl-Lösung gewaschen, mit $MgSO_4$ getrocknet und eingedampft. Nach Chromatographie des Rohprodukt an Kieselgel mit Hexan/Essigsäureäthylester 2:1 konnten 40 mg Produkt, als Epimerengemisch an C(13) (13β:13α ca. 9:1) isoliert werden.
MS: (m/e): 570 ($M^+$, $C_{33}H_{46}O_8$)
13β-Formyl-milbemycin $A_4$:

$^1H$-NMR (300 MHz, $CDCl_3$):
2,83 ppm (m) ($C_{12}H$)
2,91 ppm (dd, $J_1 = 2$, $J_2 = 11$) ($C_{13}H$)
3,06 ppm (dt, $J_d = 2,5$, $J_t = 9$) ($C_{25}H$)
4.07 ppm (s) (OH)
5,30 ppm (t, J = 7) ($C_5H$)
9,59 ppm (d, J = 2) (CHO)

13α-Formyl-milbemycin $A_4$:
$^1H$-NMR (300 MHz, $CDCl_3$):
2,62 ppm (m) ($C_{12}H$)
3,00 ppm (bs, $W_{1/2} = 10$) ($C_{13}H$)
3,06 ppm (dt, $J_d = 2,5$, $J_t = 9$) ($C_{25}H$)
4,10 ppm (s) (OH)
9,75 ppm (bs, $W_{1/2} = 4$) (CHO)

b) Eine Lösung von 20 mg 5-O-tert.-Butyldimethylsilyl-13-formylmilbemycin $A_4$ (Epimerengemisch an C(13) (13β:13α ca. 2:1) in 1,0 ml einer $HF_x$•Pyridin/THF-Lösung (vgl. oben) wurde 16 Std. bei Raumtemperatur gerührt. Das Reaktionsgemisch wurde dann auf 50 ml ges. $NaHCO_3$-Lösung gegossen und mit 100 ml Diäthyläther extrahiert. Die organische Phase wurde mit 50 ml ges. NaCl-Lösung gewaschen, mit $MgSO_4$ getrocknet und eingedampft. Die Chromatographie des Roprodukts an Kieselgel mit Hexan/Essigsäureäthylester 2:1 ergab 16 mg 13-Formyl-milbemycin $A_4$ als Epimerengemisch an C(13) (13β:13α ca. 2:1).

Analog zu den Beispielen B1 und B2 werden die nachfolgenden Verbindungen hergestellt:
5-0-tert.-Butyldimethylsilyl-13-formyl-milbemycin $A_3$, 5-O-tert.-Butyldimethylsilyl-13-formyl-milbemycin D
13-Formyl-milbemycin $A_3$ und 13-Formyl-milbemycin D

## Herstellung von Verbindungen der Formel I

### H1. Herstellung von 5-O-tert.-Butyldimethylsilyl-13-vinyl-milbemycin $A_4$

Eine Lösung von 103 mg 5-O-tert.-Butyldimethylsilyl-13-formyl-milbemycin $A_4$ (Epimerengemisch: 13β:13α ca. 2:1) in 1,5 ml Toluol und 0,5 mg Tetrahydrofuran (THF) wurde mit 10 µl Pyridin versetzt und dann auf -40°C abgekühlt. Nach der Zugabe von 1,20 ml einer 0,5 M Lösung von $Cp_2TiCH_2AlCl(CH_3)_2$ (V) in Toluol wurde 1 Std. im Temperaturbereich von -40° bis -30°C unter Argon gerührt, dann das Reaktionsgemisch mit 160 µl 15 % NaOH-Lösung versetzt und langsam auf 0°C aufgewärmt. Das Reaktionsgemisch wurde mit 50 ml Diäthyläther verdünnt, mit $MgSO_4$ getrocknet, mit Hilfe von Celite filtriert und eingedampft. Nach Chromatographie des Rohprodukts an Kieselgel mit Hexan/Essigsäureäthylester 10:1 konnten neben 15 mg Edukt 84 mg Produkt als Epimerengemisch an C(13) (13β:13α ca. 2:1) isoliert werden.
MS: (m/e): 682 ($M^+$, $C_{40}H_{62}O_7Si$)

### H2. Herstellung von 5-O-tert.-Butyldimethylsilyl-13β-vinyl-milbemycin $A_4$

Analog Herstellungsbeispiel H1 konnten aus 107 mg 5-O-tert.-Butyldimethylsilyl-13β-formyl-milbemycin $A_4$ und 1,26 ml einer 0,5 M Lösung von $Cp_2TiCH_2AlCl(CH_3)_2$ (V) in Toluol neben 17 mg Edukt 83 mg Produkt erhalten werden.
MS: (m/e): 682 ($M^+$, $C_{40}H_{62}O_7Si$)
$^1H$-NMR (300 MHz, $CDCl_3$):
2,45 ppm (dd, $J_1 = 9$, $J_2 = 10$) ($C_{13}H$)
3,04 ppm (dt, $J_d = 2,5$, $J_t = 9$) ($C_{25}H$)
4,00 ppm (s) (OH)
4,42 ppm (bs, $W_{1/2} = 10$) ($C_5H$)
5,03 ppm (dd, $J_1 = 1$, $J_2 = 17$) ($\underline{H}HC=CH$, trans)
5,05 ppm (dd, $J_1 = 1$, $J_2 = 11$) ($H\underline{H}C=CH$, cis)
5,80 ppm (m) ($HHC=C\underline{H}$)

### H3. Herstellung von 5-O-tert.-Butyldimethylsilyl-13α-vinyl-milbemycin $A_4$

Aus 91 mg 5-O-tert.-Butyldimethylsilyl-13α-formyl-milbemycin $A_4$ und 1,07 ml einer 0,5 M Lösung von $Cp_2TiCH_2AlCl(CH_3)_2$ (V) in Toluol konnten analog Herstellungsbeispiel H1 76 mg Produkt erhalten werden.
MS: (m/e): 682 ($M^+$, $C_{40}H_{62}O_7Si$)
$^1H$-NMR (300 MHz, $CDCl_3$):
2,63 ppm (bd, J = 9) ($C_{13}H$)
3,05 ppm (dt, $J_d = 2,5$, $J_t = 10$) ($C_{25}H$)
4,14 ppm (s) (OH)
4,41 ppm (bs, $W_{1/2} = 10$) ($C_5H$)
5,04 ppm (dd, $J_1 = 2$, $J_2 = 17$) ($\underline{H}HC=CH$, trans)
5,18 ppm (dd, $J_1 = 2$, $J_2 = 10$) ($H\underline{H}C=CH$, cis)
5,93 ppm (dt, $J_d = 17$, $J_t = 10$) ($HHC=C\underline{H}$)

H4. Herstellung von 13-Vinyl-milbemycin $A_4$

Eine Lösung von 41 mg 5-O-tert.-Butyldimethylsilyl-13-vinyl-milbemycin $A_4$ (Epimerengmisch: 13β:13α ca. 2:1) in 1,0 ml einer 5 % Lösung von 40 % wässrigem HF in Acetonitril wurde 1 Std. bei Raumtemperatur gerührt, dann auf 50 ml ges. $NaHCO_3$-Lösung gegossen und mit 100 ml Diäthyläther extrahiert. Die organische Phase wurde mit 50 ml ges. NaCl-Lösung gewaschen, mit $MgSO)_4$ getrocknet und eingedampft. Nach Chromatographie des Roprodukts an Kieselgel mit Hexan/Essigsäureäthylester 2:1 konnten 32 mg Produkt als Epimerengemisch an C(13) (13β:13α ca. 2:1) isoliert werden.
MS: (m/e): 568 ($M^+$, $C_{34}H_{48}O_7$)

H5. Herstellung von 13β-Vinyl-milbemycin $A_4$

Eine Lösung von 79 mg 5-O-tert.-Butyldimethylsilyl-13β-vinyl-milbemycin $A_4$ in 1,0 ml einer $HF_x$•Pyridin/THF-Lösung (vgl. oben) wurde 17 Std. bei Raumtemperatur gerührt, dann auf 50 ml ges. $NaHCO_3$-Lösung gegossen und mit 100 ml Diäthyläther extrahiert. Die organische Phase wurde mit 50 ml ges. NaCl-Lösung gewaschen, mit $MgSO_4$ getrocknet und eingedampft. Chromatographie des Roprodukts an Kieselgel mit Hexan/Essigsäureäthylester ergab 63 mg Produkt.
MS: (m/e): 568 ($M^+$, $C_{34}H_{48}O_7$)
$^1$H-NMR (300 MHz, $CDCl_3$):
2,47 ppm (dd, $J_1$ = 9, $J_2$ = 10) ($C_{13}H$)
3,07 ppm (dt, $J_d$ = 2,5, $J_t$ = 9) ($C_{25}H$)
4,01 ppm (s) (OH)
4,28 ppm (t, J = 7) ($C_5H$)
5,03 ppm (dd, $J_1$ = 1, $J_2$ = 17) (HHC=CH, trans)
5,05 ppm (dd, $J_1$ = 1, $J_2$ = 11) (HHC=CH, cis)
5,79 ppm (m) (HHC=CH)

H6. Herstellung von 5-O-tert.-Butyldimethylsilyl-13-vinylmilbemycin $A_3$

Analog Herstellungsbeispiel H1 erhält man aus 50 mg 5-O-tert.-Butyldimethylsilyl-13-formyl-milbemycin $A_3$ (Epimerengemisch: 13β:13α ca. 2:1) und 0,75 ml einer 0,5 M Lösung von $Cp_2TiCH_2AlCl(CH_3)_2$ (V) die Titelverbindung als Epimerengemisch an C(13) (13β:13α ca. 2:1).
MS: (m/e): 668 ($M^+$, $C_{39}H_{60}O_7Si$)

H7. Herstellung von 13-Vinyl-milbemycin $A_3$

Aus 22 mg 5-O-tert.-Butyldimethylsilyl-13-vinyl-milbemycin $A_3$ (Epimerengemisch: 13β:13α ca. 2:1) und 1,0 ml einer $HF_x$• Pyridin/THF-Lösung kann die Titelverbindung (Epimerengemisch: 13β:13α ca. 2:1) analog Herstellungbeispiel H4 gewonnen werden.
MS: (m/e): 554 ($M^+$, $C_{33}H_{46}O_7$)

H8. Herstellung von 5-O-Chloracetyl-13-vinyl-milbemycin $A_4$

Eine Lösung von 35 mg 13-Vinyl-milbemycin $A_4$ (Epimerengemisch: 13β:13α ca. 2:1) und 50 µl Pyridin in 2 ml Methylenchlorid wird bei 0° C mit 10 µl Chloracetylchlorid versetzt. Nach einer Stunde Rühren bei 0° C werden 100 ml Diethylether zugegeben. Die erhaltene Lösung wird mit 1N HCl gewaschen, über $MgSO_4$ getrocknet und eingedampft. Die Chromatographie des Rohprodukts an Kieselgel mit Hexan/Essigsäureäthylester 4:1 ergibt die Titelverbindung als Epimerengemisch an C(13) (13β:13α ca. 2:1).
MS: (m/e): 644 ($M^+$, $C_{36}H_{49}O_8$)

H9. Herstellung von 5-O-Acetoxy-acetyl-13-vinyl-milbemycin $A_4$

Zu einer Lösung von 25 mg 13-Vinyl-milbemycin $A_4$ (Epimerengemisch: 13β:13α ca. 2:1), 40 µl Pyridin und 1 mg Dimethylaminopyridin in 1 ml Methylenchlorid wird bei 0°C 1 ml einer 0,09 M Lösung von Acetoxyacetylchlorid in Benzol gegeben. Nach 2 Stunden Rühren bei 0°C wird das Reaktionsgemisch zwischen Diethylether und 1N HCl verteilt. Die organische Phase wird mit $MgSO_4$ getrocknet und eingedampft. Die Chromatographie des Rohproduktes an Kieselgel mit Hexan/Essigsäureäthylester 3:1 ergibt die Titelverbindung als Epimerengemisch an C(13) (13β:13α ca. 2:1).
MS: (m/e): 668 ($M^+$, $C_{38}H_{52}O_{10}$)

H10. Herstellung von 5-O-(1,2,4-Triazol-1-yl)-acetyl-13-vinylmilbemycin $A_4$

Zu einer Lösung von 32 mg 13-Vinyl-milbemycin $A_4$ (Epimerengemisch: 13β:13α ca. 2:1) und 20 mg 1H-1,2,4-Triazol-1-yl-essigsäure in 2 ml Tetrahydrofuran werden 0,1 ml Pyridin, 1 mg Dimethylaminopyridin und 25 mg N,N-Dicyclohexylcarbodiimid gegeben. Das Reaktionsgemisch wird anschliessend 3 Stunden bei Raumtemperatur gerührt und dann einge dampft. Das erhaltene Rohprodukt wird an Kieselgel mit Methylenchlorid/Methanol (5 %) chromatographiert und man erhält die Titelverbindung als Epimerengemisch an C(13) (13β:13α ca 2:1).
MS: (m/e): 677 ($M^+$, $C_{38}H_{51}N_3O_8$)

Analog zu den beschriebenen Arbeitsweisen werden auch die nachfolgend genannten Verbindungen der Formel I hergestellt; wobei die nachfolgenden Tabellen keinen limitierenden Charakter haben:

Tabelle I: Typische Vertreter von Verbindungen der Formel I

| Verb. Nr. | R | $OR_1$ |
|---|---|---|
| 1.1 | $CH_3$ | OH |
| 1.2 | $C_2H_5$ | OH |
| 1.3 | $C_3H_7$-iso | OH |
| 1.4 | $C_4H_9$-sec. | OH |
| 1.5 | $CH_3$ | $OSi(CH_3)_3$ |
| 1.6 | $C_2H_5$ | $OSi(CH_3)_3$ |
| 1.7 | $C_3H_7$-iso | $OSi(CH_3)_3$ |
| 1.8 | $C_4H_9$-sec. | $OSi(CH_3)_3$ |
| 1.9 | $CH_3$ | $OSi(t\text{-}C_4H_9)(CH_3)_2$ |
| 1.10 | $C_2H_5$ | $OSi(t\text{-}C_4H_9)(CH_3)_2$ |
| 1.11 | $C_3H_7$-iso | $OSi(t\text{-}C_4H_9)(CH_3)_2$ |
| 1.12 | $C_4H_9$-sec. | $OSi(t\text{-}C_4H_9)(CH_3)_2$ |
| 1.13 | $CH_3$ | $OCOCH_3$ |
| 1.14 | $C_2H_5$ | $OCOCH_3$ |
| 1.15 | $C_3H_7$-iso | $OCOCH_3$ |
| 1.16 | $C_4H_9$-sec. | $OCOCH_3$ |
| 1.17 | $CH_3$ | $OCOCH_2Cl$ |
| 1.18 | $C_2H_5$ | $OCOCH_2Cl$ |
| 1.19 | $C_3H_7$-iso | $OCOCH_2Cl$ |
| 1.20 | $C_4H_9$-sec. | $OCOCH_2Cl$ |
| 1.21 | $CH_3$ | $OCOCH_2Br$ |
| 1.22 | $C_2H_5$ | $OCOCH_2Br$ |
| 1.23 | $C_3H_7$-iso | $OCOCH_2Br$ |
| 1.24 | $C_4H_9$-sec. | $OCOCH_2Br$ |
| 1.25 | $CH_3$ | $OCOCH_2F$ |
| 1.26 | $C_2H_5$ | $OCOCH_2F$ |
| 1.27 | $C_3H_7$-iso | $OCOCH_2F$ |
| 1.28 | $C_4H_9$-sec. | $OCOCH_2F$ |
| 1.29 | $CH_3$ | $OCOCH_2BrF$ |
| 1.30 | $C_2H_5$ | $OCOCH_2BrF$ |
| 1.31 | $C_3H_7$-iso | $OCOCH_2BrF$ |
| 1.32 | $C_4H_9$-sec. | $OCOCH_2BrF$ |
| 1.33 | $CH_3$ | $OCOCH_2OCH_3$ |
| 1.34 | $C_2H_5$ | $OCOCH_2OCH_3$ |
| 1.35 | $C_3H_7$-iso | $OCOCH_2OCH_3$ |
| 1.36 | $C_4H_9$-sec. | $OCOCH_2OCH_3$ |

Tabelle I: (Fortsetzung)

| Verb. Nr. | R | $OR_1$ |
|---|---|---|
| 1.37 | $CH_3$ | $OCOCH_2SCH_3$ |
| 1.38 | $C_2H_5$ | $OCOCH_2SCH_3$ |
| 1.39 | $C_3H_7$-iso | $OCOCH_2SCH_3$ |
| 1.40 | $C_4H_9$-sec. | $OCOCH_2SCH_3$ |
| 1.41 | $CH_3$ | $OCOCH_2OH$ |
| 1.42 | $C_2H_5$ | $OCOCH_2OH$ |
| 1.43 | $C_3H_7$-iso | $OCOCH_2OH$ |
| 1.44 | $C_4H_9$-sec. | $OCOCH_2OH$ |
| 1.45 | $CH_3$ | $OCOCH_2OSi(t\text{-}C_4H_9)(CH_3)_2$ |
| 1.46 | $C_2H_5$ | $OCOCH_2OSi(t\text{-}C_4H_9)(CH_3)_2$ |
| 1.47 | $C_3H_7$-iso | $OCOCH_2OSi(t\text{-}C_4H_9)(CH_3)_2$ |
| 1.48 | $C_4H_9$-sec. | $OCOCH_2OSi(t\text{-}C_4H_9)(CH_3)_2$ |
| 1.49 | $CH_3$ | $OCOCH_2OCH_2OCH_3$ |
| 1.50 | $C_2H_5$ | $OCOCH_2OCH_2OCH_3$ |
| 1.51 | $C_3H_7$-iso | $OCOCH_2OCH_2OCH_3$ |
| 1.52 | $C_4H_9$-sec. | $OCOCH_2OCH_2OCH_3$ |
| 1.53 | $CH_3$ | $OCOCH_2O$— 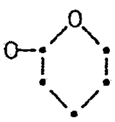 |
| 1.54 | $C_2H_5$ | $OCOCH_2O$— 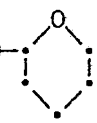 |
| 1.55 | $C_3H_7$-iso | $OCOCH_2O$— 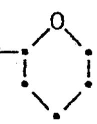 |
| 1.56 | $C_4H_9$-sec. | $OCOCH_2O$— 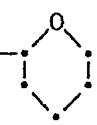 |
| 1.57 | $CH_3$ | $OCOCH_2OSO_2\text{-}C_6H_4(CH_3)(4)$ |
| 1.58 | $C_2H_5$ | $OCOCH_2OSO_2\text{-}C_6H_4(CH_3)(4)$ |
| 1.59 | $C_3H_7$-iso | $OCOCH_2OSO_2\text{-}C_4H_9(CH_3)(4)$ |
| 1.60 | $C_4H_9$-sec. | $OCOCH_2OSO_2\text{-}C_4H_9(CH_3)(4)$ |
| 1.61 | $CH_3$ | $OCOCH_2OSO_2CH_3$ |
| 1.62 | $C_2H_5$ | $OCOCH_2OSO_2CH_3$ |
| 1.63 | $C_3H_7$-iso | $OCOCH_2OSO_2CH_3$ |
| 1.64 | $C_4H_9$-sec. | $OCOCH_2OSO_2CH_3$ |

Tabelle I: (Fortsetzung)

| Verb. Nr. | R | $OR_1$ |
|---|---|---|
| 1.65 | $CH_3$ | $OCOCH_2OCOCH_3$ |
| 1.66 | $C_2H_5$ | $OCOCH_2OCOCH_3$ |
| 1.67 | $C_3H_7$-iso | $OCOCH_2OCOCH_3$ |
| 1.68 | $C_4H_9$-sec. | $OCOCH_2OCOCH_3$ |
| 1.69 | $CH_3$ | $OCOCH_2OCO$— 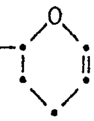 |
| 1.70 | $C_2H_5$ | $OCOCH_2OCO$— 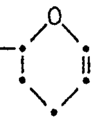 |
| 1.71 | $C_3H_7$-iso | $OCOCH_2OCO$— 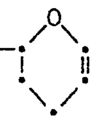 |
| 1.72 | $C_4H_9$-sec. | $OCOCH_2OCO$— 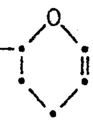 |
| 1.73 | $CH_3$ | $OCOCH_2OCO-C_6H_4Cl(3)$ |
| 1.74 | $C_2H_5$ | $OCOCH_2OCO-C_6H_4Cl(3)$ |
| 1.75 | $C_3H_7$-iso | $OCOCH_2OCO-C_4H_9Cl(3)$ |
| 1.76 | $C_4H_9$-sec. | $OCOCH_2OCO-C_4H_9CH_3(3)$ |
| 1.77 | $CH_3$ | $OCOCH_2OCO$-Thiophen-1-yl |
| 1.78 | $C_2H_5$ | $OCOCH_2OCO$-Thiophen-1-yl |
| 1.79 | $C_3H_7$-iso | $OCOCH_2OCO$-Thiophen-1-yl |
| 1.80 | $C_4H_9$-sec. | $OCOCH_2OCO$-Thiophen-1-yl |
| 1.81 | $CH_3$ | $OCOCH_2OCOCH_2F$ |
| 1.82 | $C_2H_5$ | $OCOCH_2OCOCH_2F$ |
| 1.83 | $C_3H_7$-iso | $OCOCH_2OCOCH_2F$ |
| 1.84 | $C_4H_9$-sec. | $OCOCH_2OCOCH_2F$ |
| 1.85 | $CH_3$ | $OCOCH_2OCOCH_2CH_2CH_2Cl$ |
| 1.86 | $C_2H_5$ | $OCOCH_2OCOCH_2CH_2CH_2Cl$ |
| 1.87 | $C_3H_7$-iso | $OCOCH_2OCOCH_2CH_2CH_2Cl$ |
| 1.88 | $C_4H_9$-sec. | $OCOCH_2OCOCH_2CH_2CH_2Cl$ |
| 1.89 | $CH_3$ | $OCOCH_2OC_6H_5$ |
| 1.90 | $C_2H_5$ | $OCOCH_2OC_6H_5$ |
| 1.91 | $C_3H_7$-iso | $OCOCH_2OC_6H_5$ |
| 1.92 | $C_4H_9$-sec. | $OCOCH_2OC_6H_5$ |

Tabelle I: (Fortsetzung)

| Verb. Nr. | R | $OR_1$ |
|---|---|---|
| 1.93 | $CH_3$ | $OCOCH_2OCOOCH_3$ |
| 1.94 | $C_2H_5$ | $OCOCH_2OCOOCH_3$ |
| 1.95 | $C_3H_7$-iso | $OCOCH_2OCOOCH_3$ |
| 1.96 | $C_4H_9$-sec. | $OCOCH_2OCOOCH_3$ |
| 1.97 | $CH_3$ | $OCOCH_2$-1H-Triazol-1-yl |
| 1.98 | $C_2H_5$ | $OCOCH_2$-1H-Triazol-1-yl |
| 1.99 | $C_3H_7$-iso | $OCOCH_2$-1H-Triazol-1-yl |
| 1.100 | $C_4H_9$-sec. | $OCOCH_2$-1H-Triazol-1-yl |
| 1.101 | $CH_3$ | $OCOCH_2$-1H-Triazol-1-yl 1/2 $ZnCl_2$ |
| 1.102 | $C_2H_5$ | $OCOCH_2$-1H-Triazol-1-yl 1/2 $ZnCl_2$ |
| 1.103 | $C_3H_7$-iso | $OCOCH_2$-1H-Triazol-1-yl 1/2 $ZnCl_2$ |
| 1.104 | $C_4H_9$-sec. | $OCOCH_2$-1H-Triazol-1-yl 1/2 $ZnCl_2$ |
| 1.105 | $CH_3$ | $OCOCH_2OCO$– 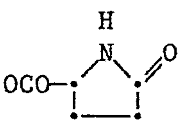 |
| 1.106 | $C_2H_5$ | $OCOCH_2OCO$– 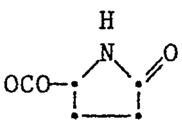 |
| 1.107 | $C_3H_7$-iso | $OCOCH_2OCO$– 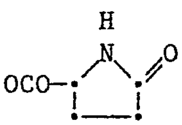 |
| 1.108 | $C_4H_9$-sec. | $OCOCH_2OCO$– 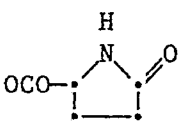 |
| 1.109 | $CH_3$ | $OCOC_5H_{12}$-n |
| 1.110 | $C_2H_5$ | $OCOC_5H_{12}$-n |
| 1.111 | $C_3H_7$-iso | $OCOC_5H_{12}$-n |
| 1.112 | $C_4H_9$-sec. | $OCOC_5H_{12}$-n |

Formulierungsbeispiele für den Wirkstoff der Formel I (% = Gewichtsprozent)

| Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Emulsions-KonzentratWirkstoff aus den Tabellen 10 %
Octylphenolpolyethylenglykolether (4-5 Mol AeO) 3 %
Ca-Dodecylbezolsulfonat 3 %
Ricinusölpolyglykolether (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder GranulatWirkstoff aus den Tabellen 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

Tabletten bzw. Boli

I Ein Wirkstoff aus den Tabellen 33,0 %
Methylcellulose 0,80 %
Kieselsäure hochdispers 0,80 %
Maisstärke 8,40 %

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

II Milchzucker krist. 22,50 %
Maisstärke 17,00 %
mikrokrist. Cellulose 16,50 %
Magnesiumstearat 1,00 %
Alle 4 Hilfsstoffe gut mischen
Phasen I und II mischen und zu Tabletten oder Boli verpressen.

Injektabiles

A. Oeliges Vehikel (langsame Freisetzung)
Ein Wirkstoff aus den Tabellen 0,1-1,0 g
Erdnussöl ad 100 ml

Ein Wirkstoff aus den Tabellen 0,1-1,0 g
Sesamöl ad 100 ml
Herstellung: Der Wirkstoff wird in einem Teil des Oels unter Rühren und gegebenenfalls leichtem Erwärmen gelöst, nach Abkühlung auf das Sollvolumen aufgefüllt und durch ein geeignetes Membranfilter mit 0,22 µm sterilfiltriert.

B. Wassermischbares Lösungsmittel (mittlere Freisetzungsgeschwindigkeit)
Ein Wirkstoff aus den Tabellen 0,1-1,0 g
4-Hydroxymethyl-1,3-dioxolan (Glycerol Formal) 40 g
1,2-Propandiol ad 100 ml

Ein Wirkstoff aus den Tabellen 0,1-1,0 g
Glycerindimethylketal 40 g
1,2-Propandiol ad 100 ml
Herstellung: Der Wirkstoff wird in einem Teil des Lösungsmittels unter Rühren gelöst, auf des Sollvolumen aufgefüllt und durch ein geeignetes Membranfilter mit 0.22 µm sterilfiltriert.

C. Wässriges Solubilisat (rasche Freisetzung)
Ein Wirkstoff aus den Tabellen 0,1-1,0 g
Polyäthoxyliertes Ricinusöl (40 Aethylenoxideinheiten)* 10 g
1,2-Propandiol 20 g
Benzylalkohol 1 g.
Aqua ad injekt. ad 100 ml
* Im Handel erhältlich unter der Bezeichnung CREMOPHOR® EL (BASF AG);
Ein Wirkstoff aus den Tabellen 0,1-1,0 g
Polyäthoxyliertes Sorbitanmonooleat (20 Aethylenoxideinheiten)** 8 g
4-Hydroxymethyl-1,3-dioxolan (Glycerol Formal) 20 g
Benzylalkohol 1 g
Aqua ad injekt. ad 100 ml
** Im Handel erhältlich unter der Bezeichnung TWEEN® 80 (ICI);
Herstellung: Der Wirkstoff wird in den Lösungsmitteln und dem Tensid gelöst und mit Wasser auf das Sollvolumen aufgefüllt. Sterilfiltration durch geeignetes Membranfilter mit 0,22 µm Porendurchmesser.
Die wässrigen Systeme können bevorzugterweise auch für die orale und/oder intraruminale Applikation eingesetzt werden.

Biologische Beispiele

B-1. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50° C vermischt, dass ein Homogenisat von wahlweise 250 ppm oder 100 ppm Wirkstoffgehalt entsteht. In jede Reagenzglas-Probe werden ca. 30 Lucilia-Larven ($L_1$) eingesetzt. Nach 4 Taben wird die Mortalitätsrate bestimmt. Verbindungen der Formel I aus den Tabellen, wie z.B. die Verbindungen H4 und H5 erzielten mit 100 ppm eine Wirkung von 100 %.

B-2. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von wahlweise 1, 0,1 oder 0,01 µg pro Zecke gelöst ist. Kontrolltiere erhalten eine wirkstofffreie Injection. Nach der Behandlung werden die Tiere unter Normalbedingungen in einem Insektarium bei ca. 28° C und 80 % relativer Luftfeuchtigkeit gehalten, bis die Eiablage erfolgt und die Larven aus den Eiern der Kontrolltiere geschlüpft

sind.

Die Aktivität einer geprüften Substanz wird mit der $IR_{90}$ bistimmt, d.h. es wird jene Wirkstoffdosis ermittelt, bei der noch nach 30 Tagen 9 von 10 Zeckenweibchen (=90 %) Eier ablegen, die nicht schlupffähig sind.

Verbindungen der Formeln I aus den Tabellen, insbesondere jene der Herstellungsbeispiele H1 bis H5, zeigen in diesem Versuch eine sehr gute Wirkung mit einem $IR_{90}$ von 0,1 µg.

B-3. Versuch an mit Nematoden (Haemonchus contortus und Trichostrongylus colubriformis) infizierten Schafen

Der Wirkstoff wird als Suspension formuliert mit einer Magensonde oder durch Injektion in den Pansen eines Schafes gegeben, das mit Haemonchus contortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 0,5 mg oder 0,2 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit Verbindungen der Formeln I wie z.B. mit der H4 und H5 bis 0,2 mg/kg behandelt wurden, zeigten im Vergleich zu unbehandelten, aber infizierten Vergleichsgruppen keinen Nematodenbefall (= komplette Reduktion der Wurmeier im Kot).

B-4. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen der Formeln I aus den Tabellen bewirken in diesem Test bei einer Konzentration von 1.6 ppm bereits nach einem Tag eine vollständige Abtötung sämtlicher Larven.

B-5. Milbizide Wirkung gegen Dermanyssus gallinae

2 bis 3 ml einer Testlösung (100, 10, 1 und 0,1 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig, bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Milben zur Evaluierung der Wirksamkeit der Testsubstanzen drei Tage unter Laborbedingungen beobachtet, wobei die Mortalität als Massstab für Wirksamkeit herangezogen wird.

Verbindungen aus den Tabellen, wie z.B. die Verbindungen H4 und H5 zeigen bei 100 ppm eine Wirkung von 100 %.

**Patentansprüche**

1. Eine Verbindung der Formel I

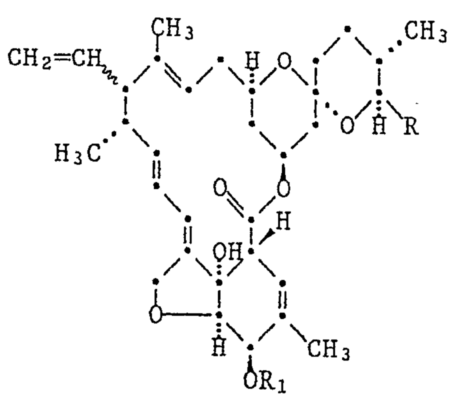

(I)

worin

R für Methyl, Ethyl, Isopropyl oder sek.-Butyl oder für die Gruppe 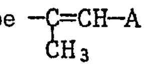 steht, wobei A Methyl, Ethyl oder Isopropyl bedeutet; und

$R_1$ Wasserstoff, eine Silyl- oder eine Acylgruppe darstellt; unter Einschluss ihrer Säureadditionssalze und

Metallkomplexe.

2. Eine Verbindung der Formel I nach Anspruch 1

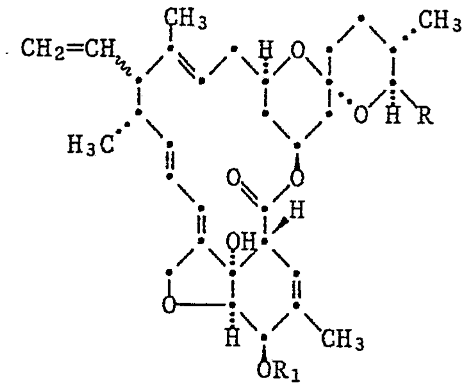

(I)

worin

R für Methyl, Ethyl, Isopropyl oder sek. -Butyl steht; und

$R_1$ Wasserstoff, eine Silyl- oder eine Acylgruppe darstellt; unter Einschluss ihrer Säureadditionssalze und Metallkomplexe.

3. Eine Verbindung der Formel I nach Anspruch 1, worin R für Methyl, Ethyl, Isopropyl oder sek.-Butyl steht; und $R_1$ Wasserstoff oder die Silylgruppe -Si($R_2$)($R_3$)($R_4$) darstellt, worin $R_2$, $R_3$ und $R_4$ unabhängig voneinander für $C_1$-$C_6$-Alkyl, Benzyl oder Phenyl stehen oder für Alkylcarbonyl, Arylcarbonyl oder Aralkylcarbonyl steht.

4. Eine Verbindung der Formel I nach Anspruch 1, worin

R für Methyl, Ethyl, Isopropyl oder sek.Butyl steht und

$R_1$ Wasserstoff darstellt.

5. Eine Verbindung der Formel I nach Anspruch 1, worin

R für Methyl, Ethyl, Isopropyl oder sek. Butyl steht und

$R_1$ eine Silylgruppe darstellt.

6. Eine Verbindung der Formel I nach Anspruch 4, worin

R für Methyl, Ethyl, Isopropyl oder sek.-Butyl steht;

$R_1$ eine der Acylgruppen

a) 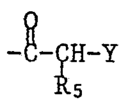 ,

b) 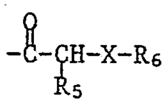 ,

c) 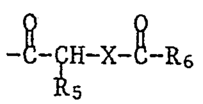 oder

d) 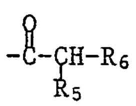

repräsentiert, wobei

X für Sauerstoff oder Schwefel steht;

Y für Halogen, Azido oder einen Sulfonsäurerest steht;

$R_5$ Wasserstoff, $C_1$-$C_4$-Alkyl oder Halogen bedeutet; und

$R_6$ für Wasserstoff, für unsubstituiertes oder durch Halogen, Hydroxy, $C_1$-$C_6$-Alkoxy oder $C_1$-$C_6$-Haloalkoxy substituiertes $C_1$-$C_{10}$-Alkyl, für einen unsubstituierten oder substituierten Rest ausgewählt aus der Reihe $C_3$-$C_{10}$-Cycloalkyl, $C_2$-$C_6$-Alkenyl und $C_3$-$C_6$-Alkinyl, wobei die Substituenten ausgewählt sind aus der Reihe Halogen, Hydroxy, $C_1$-$C_6$-Alkoxy und $C_1$-$C_6$-Alkanyloxy, für unsubstituiertes oder durch Halogen, Cyano, $C_1$-$C_3$-Alkyl, $C_1$-$C_3$-Haloalkyl, $C_1$-$C_3$-Alkoxy, $C_1$-$C_3$-Haloalkoxy und/oder Nitro substituiertes Phenyl oder für einen unsubstituierten oder substituierten, ungesättigten oder gesättigten, fünf-

oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht, unter Einschluss der Säureadditionssalze und Metallkomplexe.

7. Eine Verbindung der Formel I nach Anspruch 6, worin
R für Methyl, Ethyl, Isopropyl oder sek.-Butyl steht;
$R_1$ die Gruppe

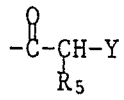

$R_5$ für Wasserstoff, Fluor oder Methyl steht; und
Y Chlor, Brom, Jod, Benzolsulfonyloxy, Paratosyloxy oder Mesyloxy bedeutet.

8. Eine Verbindung der Formel I nach Anspruch 6, worin
R für Methyl, Ethyl, Isopropyl oder sek.-Butyl steht;
$R_1$ die Gruppe

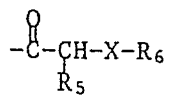

repräsentiert; wobei
X für Sauerstoff oder Schwefel steht;
$R_5$ Wasserstoff, Fluor oder Methyl bedeutet; und
$R_6$ für Wasserstoff, für unsubstituiertes oder durch Fluor, Chlor, Brom, $C_1$-$C_6$-Alkoxy, $C_3$-$C_6$-Alkoxyalkoxy, Thioalkoxy oder $C_1$-$C_3$-Haloalkoxy substituiertes $C_1$-$C_6$-Alkyl, für unsubstituiertes oder Fluor, Chlor, Brom, Methyl, Ethyl, Methoxy, Halomethoxy, Trifluormethyl und/oder Nitro substituiertes Phenyl oder Benzyl oder für einen unsubstituierten oder substituierten, ungesättigten oder gesättigten, fünf- oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht.

9. Eine Verbindung der Formel I nach Anspruch 6, worin
R für Methyl, Ethyl, Isopropyl oder sek.-Butyl steht;
$R_1$ die Gruppe

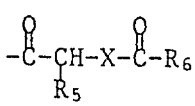

repräsentiert; wobei
X für Sauerstoff oder Schwefel steht;
$R_5$ Wasserstoff, Fluor oder Methyl bedeutet; und
$R_6$ für Wasserstoff, für unsubstituiertes oder durch Fluor, Chlor, Brom, Hydroxy, $C_1$-$C_6$-Alkoxy oder $C_1$-$C_3$-Haloalkoxy substituiertes $C_1$-$C_6$-Alkyl, für einen unsubstituierten oder substituierten Rest ausgewählt aus der Reihe $C_3$-$C_7$-Cycloalkyl, $C_2$-$C_4$-Alkenyl und $C_2$-$C_4$-Alkinyl, wobei die Substituenten ausgewählt sind aus der Reihe Fluor, Chlor, Brom, $C_1$-$C_3$-Alkoxy und $C_1$-$C_4$-Alkanoyloxy, für unsubstituiertes oder Fluor, Chlor, Brom, Methyl, Ethyl, Methoxy, Halomethoxy, Trifluormethyl und/oder Nitro substituiertes Phenyl oder für einen unsubstituierten oder substituierten, ungesättigten oder gesättigten, fünf- oder sechsgliedrigen heterocyclischen Ring mit ein bis drei Heteroatomen, ausgewählt aus der Reihe Stickstoff, Sauerstoff und Schwefel, dessen Substituenten ausgewählt sind aus der Reihe Oxo, Halogen, $C_1$-$C_4$-Alkyl, $C_1$-$C_4$-Haloalkyl und $C_1$-$C_4$-Alkoxy steht.

10. Eine Verbindung der Formel I nach Anspruch 6, worin
R für Methyl, Ethyl, Isopropyl oder sek.-Butyl steht;
$R_1$ die Gruppe

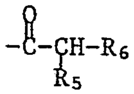

repräsentiert; wobei
$R_5$ für Wasserstoff, Fluor oder Methyl steht; und
$R_6$ für einen in 1-Position verknüpften, 5-gliedrigen Heteroaromaten mit 2 bis 3 Stickstoffatom steht, der unsubstituiert oder ein- bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

11. Eine Verbindung der Formel I nach Anspruch 6, worin
R für Methyl, Ethyl, Isopropyl oder sek.-Butyl steht;
$R_1$ eine der Acylgruppen

a) 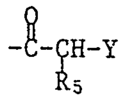 ,

b) 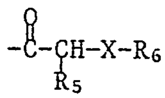 ,

c) 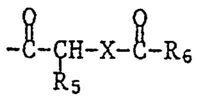 oder

d) 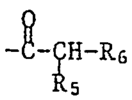

repräsentiert, wobei
X für Sauerstoff oder Schwefel steht;
Y Halogen bedeutet;
$R_5$ Wasserstoff, Halogen oder Methyl repräsentiert; und
$R_6$ für Wasserstoff, unsubstituiertes oder durch Halogen oder $C_1$-$C_4$-Alkoxy substituiertes $C_1$-$C_{14}$-Alkyl steht, für Cyclopropyl, für Cyclopentyl, für Cyclohexyl, für unsubstituiertes oder durch Fluor, Chlor, Brom, Methoxy, Trifluormethyl, Methyl und/oder Nitro substituiertes Phenyl, für 4H-2,3-Dihydropyran-2-yl oder für einen in 1-Position verknüpften, 5-gliedrigen Heteroaromaten mit 2 bis 3 Stickstoffatom steht, der unsubstituiert oder ein-bis dreifach durch $C_1$-$C_6$-Alkyl substituiert ist, steht, unter Einschluss der Säureadditionssalze mit organischen und anorganischen Säuren sowie der Metallkomplexe mit Metallkationen der ersten, zweiten, vierten oder achten Nebengruppe des Periodensystems der Elemente.

12. Eine Verbindung der Formel I nach Anspruch 1, ausgewählt aus der Reihe:
-13β-Vinyl-milbemycin D,
-13β-Vinyl-milbemycin $A_4$,
-13β-Vinyl-milbemycin $A_3$,
-13α-Vinyl-milbemycin D,
-13α-Vinyl-milbemycin $A_4$,
-13α-Vinyl-milbemycin $A_3$,

5-O-tert.-Butyldimethylsilyl-13β-vinyl-milbemycin D,
5-O-tert.-Butyldimethylsilyl-13β-vinyl-milbemycin $A_4$,
5-O-tert.-Butyldimethylsilyl-13β-vinyl-milbemycin $A_3$,
5-O-tert.-Butyldimethylsilyl-13α-vinyl-milbemycin D,
5-O-tert.-Butyldimethylsilyl-13α-vinyl-milbemycin $A_4$,
5-O-tert.-Butyldimethylsilyl-13α-vinyl-milbemycin $A_3$,

5-O-Chloracetyl-13β-vinyl-milbemycin D,
5-O-Chloracetyl-13β-vinyl-milbemycin $A_4$,
5-O-Chloracetyl-13β-vinyl-milbemycin $A_3$,

5-O-Chloracetyl-13α-vinyl-milbemycin D,
5-O-Chloracetyl-13α-vinyl-milbemycin $A_4$,
5-O-Chloracetyl-13α-vinyl-milbemycin $A_3$,

5-O-Acetoxyacetyl-13β-vinyl-milbemycin D,
5-O-Acetoxyacetyl-13β-vinyl-milbemycin $A_4$,
5-O-Acetoxyacetyl-13β-vinyl-milbemycin $A_3$,
5-O-Acetoxyacetyl-13α-vinyl-milbemycin D,
5-O-Acetoxyacetyl-13α-vinyl-milbemycin $A_4$,
5-O-Acetoxyacetyl-13α-vinyl-milbemycin $A_3$,

5-O-Methoxyacetyl-13β-vinyl-milbemycin D,
5-O-Methoxyacetyl-13β-vinyl-milbemycin $A_4$,
5-O-Methoxyacetyl-13β-vinyl-milbemycin $A_3$,
5-O-Methoxyacetyl-13α-vinyl-milbemycin D,
5-O-Methoxyacetyl-13α-vinyl-milbemycin $A_4$,
5-O-Methoxyacetyl-13α-vinyl-milbemycin $A_3$,

5-O-[3-Chlorobenzoyloxy]acetyl-13β-vinyl-milbemycin D,
5-O-[3-Chlorobenzoyloxy]acetyl-13β-vinyl-milbemycin $A_4$,
5-O-[3-Chlorobenzoyloxy]acetyl-13β-vinyl-milbemycin $A_3$,
5-O-[3-Chlorobenzoyloxy]acetyl-13α-vinyl-milbemycin D,
5-O-[3-Chlorobenzoyloxy]acetyl-13α-vinyl-milbemycin $A_4$,
5-O-[3-Chlorobenzoyloxy]acetyl-13α-vinyl-milbemycin $A_3$,

5-O-[3,4-Dihydro-2H-pyran-2-yl]carboxyacetyl-13β-vinyl-milbemycin D,
5-O-[3,4-Dihydro-2H-pyran-2-yl]carboxyacetyl-13β-vinyl-milbemycin $A_4$,
5-O-[3,4-Dihydro-2H-pyran-2-yl]carboxyacetyl-13β-vinyl-milbemycin $A_3$,
5-O-[3,4-Dihydro-2H-pyran-2-yl]carboxyacetyl-13α-vinyl-milbemycin D,
5-O-[3,4-Dihydro-2H-pyran-2-yl]carboxyacetyl-13α-vinyl-milbemycin $A_4$,
5-O-[3,4-Dihydro-2H-pyran-2-yl]carboxyacetyl-13α-vinyl-milbemycin $A_3$,

5-O-[1,2,4-Triazol-1-yl]acetyl-13β-vinyl-milbemycin D,
5-O-[1,2,4-Triazol-1-yl]acetyl-13β-vinyl-milbemycin $A_4$,
5-O-[1,2,4-Triazol-1-yl]acetyl-13β-vinyl-milbemycin $A_3$,
5-O-[1,2,4-Triazol-1-yl]acetyl-13α-vinyl-milbemycin D,
5-O-[1,2,4-Triazol-1-yl]acetyl-13α-vinyl-milbemycin $A_4$ und
5-O-[1,2,4-Triazol-1-yl]acetyl-13α-vinyl-milbemycin $A_3$.

13. Verfahren zur Herstellung einer Verbindung der Formel I,

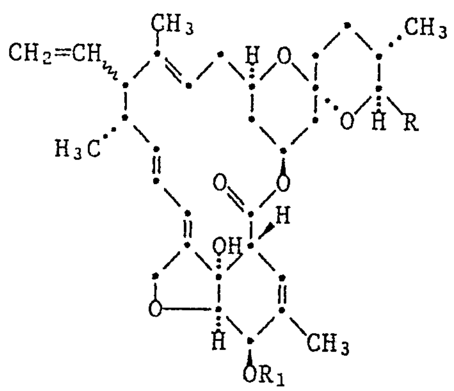

(I)

worin
R für Methyl, Ethyl, Isopropyl oder sek.-Butyl oder für die Gruppe 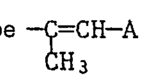 steht, wobei A Methyl, Ethyl oder Isopropyl bedeutet; und

$R_1$ Wasserstoff, eine Silyl- oder eine Acylgruppe darstellt; unter Einschluss ihrer Säureadditionssalze und Metallkomplexe, dadurch gekennzeichnet dass man eine Verbindung der Formel III

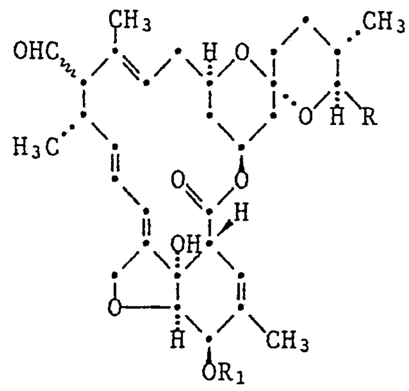

(III)

worin R für Methyl, Ethyl, Isopropyl oder sek.-Butyl oder für die Gruppe $-\underset{CH_3}{C}=CH-A$ steht, wobei A Methyl, Ethyl oder Isopropyl bedeutet, und

$R_1$ für Wasserstoff oder eine Schutzgruppe steht, mit einem zur Freisetzung des Titankomplexes der Formel IV

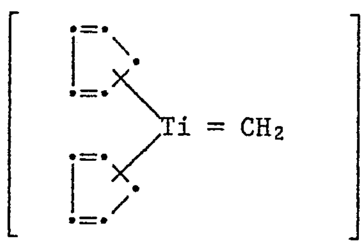

(IV)

befähigten Reagenz umsetzt und, sofern eine Verbindung der Formel I erzeugt werden soll, worin $R_1$ für eine Acylgruppe steht, eine Verbindung der Formel I, worin $R_1$ für Wasserstoff steht mit einer geeigneten Säure, deren Anhydrid oder deren Säurehalogenid verestert.

14. Verfahren nach Anspruch 13 zur Herstellung einer Verbindung der Formel I,

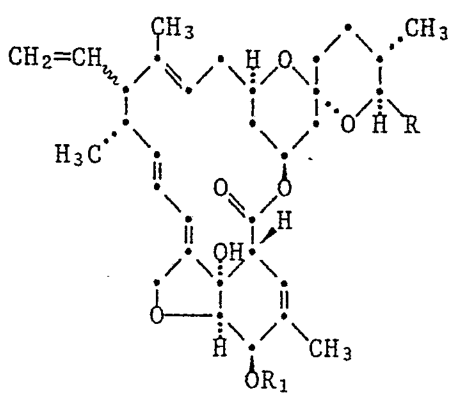

(I)

worin
R für Methyl, Ethyl, Isopropyl oder sek.-Butyl steht; und
$R_1$ Wasserstoff, eine Silyl- oder eine Acylgruppe darstellt; unter Einschluss ihrer Säureadditionssalze und Metallkomplexe, dadurch gekennzeichnet dass man eine Verbindung der Formel III

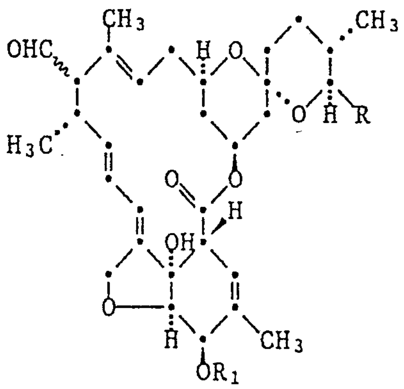

(III)

worin R für Methyl, Ethyl, Isopropyl oder sek.-Butyl steht, und $R_1$ für Wasserstoff oder eine Schutzgruppe steht, mit einem zur Freisetzung des Titankomplexes der Formel IV

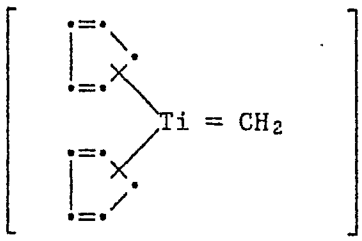

(IV)

befähigten Reagenz umsetzt und, sofern eine Verbindung der Formel I erzeugt werden soll, worin $R_1$ für eine Acylgruppe steht, eine Verbindung der Formel I, worin $R_1$ für Wasserstoff steht mit einer geeigneten Säure, deren Anhydrid oder deren Säurehalogenid verestert.

15. Mittel zur Bekämpfung von Ekto- und Endoparasiten am Nutztier oder zur Bekämpfung von Schadinsekten, dadurch gekennzeichnet, dass es neben üblichen Trägerstoffen und Verteilungsmitteln eine Verbindung der Formel I

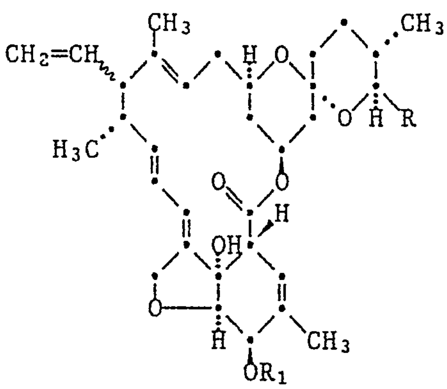

(I)

worin

R für Methyl, Ethyl, Isopropyl oder sek.-Butyl oder für die Gruppe 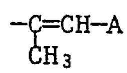 steht, wobei A Methyl, Ethyl oder Isopropyl bedeutet; und

$R_1$ Wasserstoff, eine Silyl- oder eine Acylgruppe darstellt; unter Einschluss ihrer Säureadditionssalze und Metallkomplexe, enthält.

16. Mittel zur Bekämpfung von Ekto- und Endoparasiten am Nutztier oder zur Bekämpfung von Schadinsekten nach Anspruch 15, dadurch gekennzeichnet, dass es neben üblichen Trägerstoffen und Verteilungsmitteln eine Verbindung der Formel I

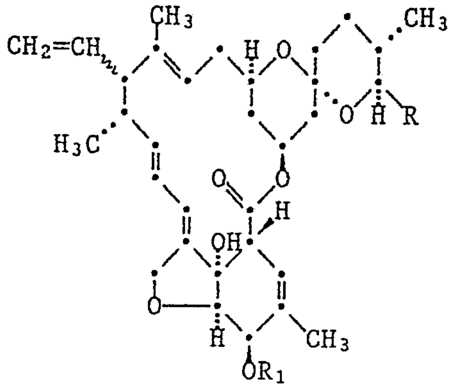

(I)

worin
R für Methyl, Ethyl, Isopropyl oder sek.-Butyl steht; und
$R_1$ Wasserstoff, eine Silyl- oder eine Acylgruppe darstellt; unter Einschluss ihrer Säureadditionssalze und Metallkomplexe, enthält.

17. Mittel nach Anspruch 15, dadurch gekennzeichnet, dass es als Wirkstoff eine Verbindung der Formel I gemäss einem der Ansprüche 3 bis 12 enthält.

18. Verfahren zur Bekämpfung von Parasiten an Tieren und Pflanzen oder von Schadinsekten, dadurch gekennzeichnet, dass man eine parasitizid bzw. insektizid wirksame Menge einer Verbindung der Formel I

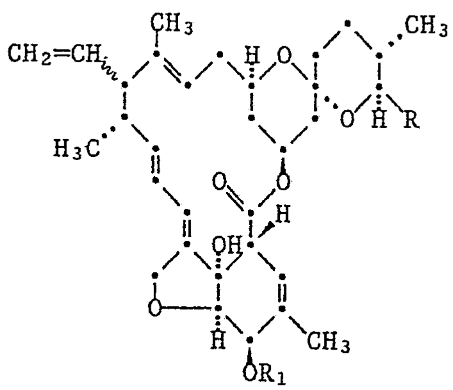

(I)

worin
R für Methyl, Ethyl, Isopropyl oder sek.-Butyl oder für die Gruppe 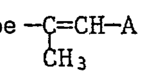 steht, wobei A Methyl, Ethyl oder Isopropyl bedeutet; und

$R_1$ Wasserstoff, eine Silyl- oder eine Acylgruppe darstellt; unter Einschluss ihrer Säureadditionssalze und Metallkomplexe in oder auf die Wirtstiere, auf die Pflanzen oder den Lebensraum der Schädlinge appliziert.

19. Verfahren zur Bekämpfung von Parasiten an Tieren und Pflanzen oder von Schadinsekten gemäss Anspruch 18, dadurch gekennzeichnet, dass man eine parasitizid bzw. insektizid wirksame Menge einer Verbindung der Formel I

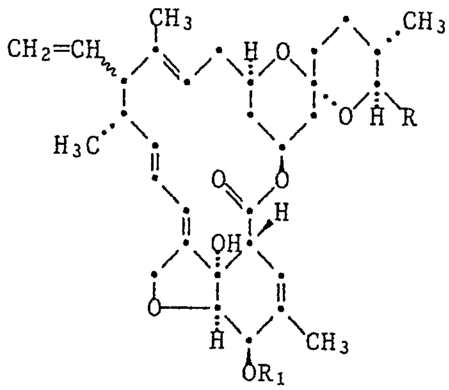

(I)

worin
R für Methyl, Ethyl, Isopropyl oder sek.-Butyl steht; und
$R_1$ Wasserstoff, eine Silyl- oder eine Acylgruppe darstellt; unter Einschluss ihrer Säureadditionssalze und Metallkomplexe in oder auf die Wirtstiere, auf die Pflanzen oder den Lebensraum der Schädlinge appliziert.

20. Verfahren nach Anspruch 18, dadurch gekennzeichnet, dass es sich bei den Parasiten um Nematoden handelt.

21. Eine Verbindunge der Formel III

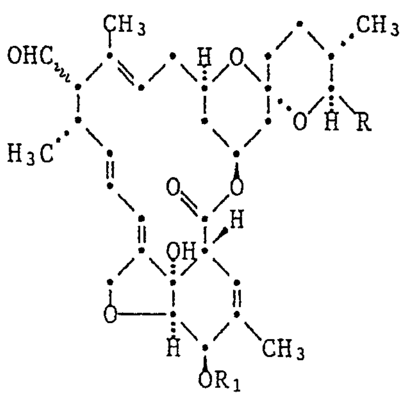

(III)

worin
R für Methyl, Ethyl, Isopropyl oder sek.-Butyl oder für eine Gruppe 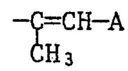 steht, wobei A Methyl, Ethyl oder Isopropyl bedeutet; und

$R_1$ Wasserstoff oder eine unter Formel I in $R_1$ definierte Schutzgruppe repräsentiert.

22. Eine Verbindung der Formel III gemäss Anspruch 21

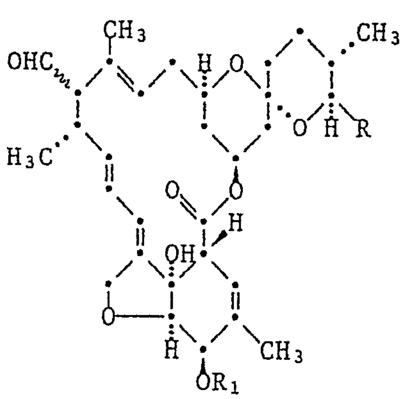

(III)

worin
R für Methyl, Ethyl, Isopropyl oder sek.-Butyl steht; und
$R_1$ Wasserstoff oder eine unter Formel I in $R_1$ definierte Schutzgruppe repräsentiert.

23. Eine Verbindung der Formel VI

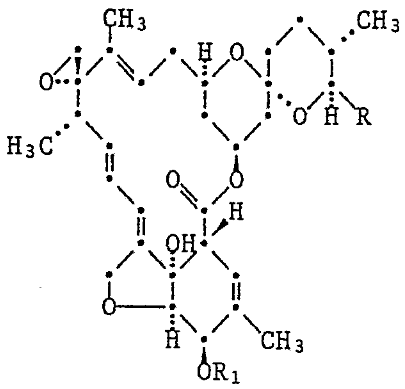

(VI)

worin
R für Methyl, Ethyl, Isopropyl oder sek.-Butyl oder für eine Gruppe 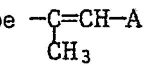 steht, wobei A Methyl, Ethyl oder Isopropyl bedeutet; und

$R_1$ Wasserstoff oder eine unter Formel I in $R_1$ definierte Schutzgruppe repräsentiert.

24. Eine Verbindung der Formel VI gemäss Anspruch 23

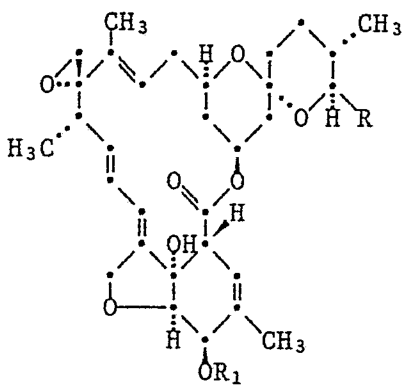

(VI)

worin
R für Methyl, Ethyl, Isopropyl oder sek.-Butyl steht; und
$R_1$ Wasserstoff oder eine unter Formel I in $R_1$ definierte Schutzgruppe repräsentiert.

25. Eine Verbindung der Formel I gemäss Anspruch 1

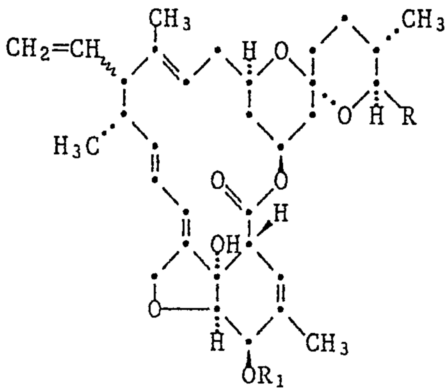

(I)

worin

R für Methyl, Ethyl, Isopropyl oder sek.-Butyl oder für die Gruppe $-\underset{CH_3}{C}=CH-A$ steht, wobei A Methyl, Ethyl oder Isopropyl bedeutet; und

$R_1$ Wasserstoff, eine Silyl- oder eine Acylgruppe darstellt; unter Einschluss ihrer Säureadditionssalze und Metallkomplexe zur Bekämpfung von Ekto- und Endoparasiten an Pflanzen und Tieren.

26. Eine Verbindung der Formel I gemäss Anspruch 2

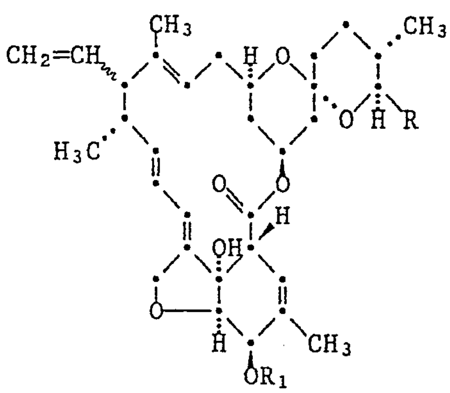

(I)

worin

R für Methyl, Ethyl, Isopropyl oder sek.-Butyl steht; und

$R_1$ Wasserstoff, eine Silyl- oder eine Acylgruppe darstellt; unter Einschluss ihrer Säureadditionssalze und Metallkomplexe zur Bekämpfung von Ekto- und Endoparasiten an Pflanzen und Tieren.

27. Eine Verbindung der Formel I gemäss einem der Ansprüche 3 bis 12 zur Bekämpfung von Ekto- und Endoparasiten an Pflanzen und Tieren.

28. Eine Verbindung der Formel I gemäss Anspruch 25 zur Bekämpfung von Endoparasiten im Warmblüter.

29. Eine Verbindung der Formel I gemäss Anspruch 26 zur Bekämpfung von Endoparasiten im Warmblüter.

30. Eine Verbindung der Formel I gemäss Anspruch 25 zur Bekämpfung von Ektoparasiten an Tieren oder von Pflanzenparasiten, die man in Aufwandmengen von 10 g bis 1000 g per Hektar auf geschlossene Kulturanbauflächen, in Pferchen, Stallungen oder sonstigen Räumen appliziert.

31. Eine Verbindung der Formel I gemäss Anspruch 26 zur Bekämpfung von Ektoparasiten an Tieren oder von Pflanzenparasiten, die man in Aufwandmengen von 10 g bis 1000 g per Hektar auf geschlossene Kulturanbauflächen, in Pferchen, Stallungen oder sonstigen Räumen appliziert.